# EUROPEAN PATENT SPECIFICATION

(11) **EP 2 431 386 B1**
(45) Date of publication and mention of the grant of the patent: **25.02.2015**
(21) Application number: 10177510.4
(22) Date of filing: 18.09.2010
(51) Int. Cl.: C07K 14/705, C12N 15/12

(54) **Na+/Mg2+ exchanger**
Na+/Mg2+-Tauscher
Échangeur de Na+/Mg2+

(43) Date of publication of application: 21.03.2012
(73) Proprietor: FBN - Leibniz-Institut für Nutztierbiologie, 18196 Dummerstorf (DE)
(72) Inventor: Röntgen, Monika, 18225 Kühlungsborn (DE); Kolisek, Martin, 14169 Berlin (DE)
(74) Representative: Wablat Lange Karthaus

(56) References cited:
- WO-A2-01/90360
- KOLISEK MARTIN ET AL: "SLC41A1 is a novel mammalian Mg2+ carrier", JOURNAL OF BIOLOGICAL CHEMISTRY, AMERICAN SOCIETY FOR BIOCHEMISTRY AND MOLECULAR BIOLOGY, INC, US, vol. 283, no. 23, 6 June 2008 (2008-06-06) , pages 16235-16247, XP002535556, ISSN: 0021-9258, DOI: DOI:10.1074/JBC.M707276200
- DATABASE UniProt [Online] 10 July 2007 (2007-07-10), "RecName: Full=Solute carrier family 41 member 1;", XP002619806, retrieved from EBI accession no. UNIPROT:Q8IVJ1 Database accession no. Q8IVJ1 -& TROELS WABAKKEN ET AL: "THE HUMAN SOLUTE CARRIER SLC41A1 BELONGS TO A NOVEL EUKARYOTIC SUBFAMILY WITH HOMOLOGY TO PROKARYOTIC MGTE MG2+ TRANSPORTERS", BIOCHEMICAL AND BIOPHYSICAL RESEARCH COMMUNICATIONS, ACADEMIC PRESS INC. ORLANDO, FL, US, vol. 306, no. 3, 4 July 2003 (2003-07-04), pages 718-724, XP008073129, ISSN: 0006-291X, DOI: DOI:10.1016/S0006-291X(03)01030-1
- SCHWEIGEL MONIKA ET AL: "Rumen epithelial cells adapt magnesium transport to high and low extracellular magnesium conditions", MAGNESIUM RESEARCH, JOHN LIBBEY, LONDON, GB, vol. 22, no. 3, 1 September 2009 (2009-09-01), pages 133-150, XP008132389, ISSN: 0953-1424
- KOLISEK MARTIN ET AL: "Cellular Mg2+ transport and homeostasis: An overview", NEW PERSPECTIVES IN MAGNESIUM RESEARCH: NUTRITION AND HEALTH SPRINGER-VERLAG LONDON LTD, SWEETAPPLE HOUSE CATTESHALL RD FARNCOMBE, GODALMING GU7 1NH, SURREY, UK, 2007, pages 21-33, XP008132427, & 11TH INTERNATIONAL MAGNESIUM SYMPOSIUM; OSAKA, JAPAN; OCTOBER 22 -26, 2006
- Anonymous: "IKMC Project Report - EUCOMM (ID: 70310)", IKMC MartSearch project website , 3 March 2010 (2010-03-03), XP002619807, Retrieved from the Internet: URL:http://www.knockoutmouse.org/martsearc h/project/70310 [retrieved on 2011-02-02]
- TADEPALLY H: "E-mail about date of availability of SLC41A1 knockout vector and mouse cells", E-MAIL, 2 February 2011 (2011-02-02), XP002619808,
- Anonymous: "HPA015138 Anti-SLC41A1 antibody produced in rabbit", Sigma-Aldrich catalog , 2009, XP002619809, Retrieved from the Internet: URL:http://www.sigmaaldrich.com/catalog/Pr oductDetail.do?lang=en&N4=HPA015138|SIGMA& N5=SEARCH_CONCAT_PNO|BRAND_KEY&F=SPEC [retrieved on 2011-01-31]
- Anonymous: "Sigma-Aldrich Antibodies 2009 Catalog", 2009, XP002619810, page 397, * L: Establishes the date of availability of the HPA015138 antibody *
- PARR CHRISTIAN ET AL: "Hepatocyte growth factor activation inhibitors (HAI-1 and HAI-2) regulate HGF-induced invasion of human breast cancer cells", INTERNATIONAL JOURNAL OF CANCER, JOHN WILEY & SONS, INC, UNITED STATES, SWITZERLAND, GERMANY, vol. 119, no. 5, 1 September 2006 (2006-09-01), pages 1176-1183, XP009141530, ISSN: 0020-7136, DOI: DOI:10.1002/IJC.21881 [retrieved on 2006-03-23]
- LI YUN FENG ET AL: "Antidepressant-like effect of agmatine and its possible mechanism.", EUROPEAN JOURNAL OF PHARMACOLOGY 23 MAY 2003 LNKD- PUBMED:12782188, vol. 469, no. 1-3, 23 May 2003 (2003-05-23), pages 81-88, XP002619811, ISSN: 0014-2999
- Anonymous: "15020", Velocigene website , 21 December 2009 (2009-12-21), XP002638499, Retrieved from the Internet: URL:http://www.velocigene.com/komp/detail/ 15020 [retrieved on 2011-05-24]
- FEIL ROBERT ET AL: "Conditional Mutagenesis: An Approach to Disease Models", HANDBOOK OF EXPERIMENTAL PHARMACOLOGY, SPRINGER VERLAG, BERLIN, DE, vol. 178, 1 January 2007 (2007-01-01), pages 3-28, XP002453073, ISSN: 0171-2004, DOI: DOI:10.1007/978-3-540-35109-2_1
- SCHWEIGEL M ET AL: "Development of monoclonal antibodies directed against the Na+/Mg2+ antiporter and their use in ruminal epithelial cells", PROCEEDINGS OF THE SOCIETY OF NUTRITION PHYSIOLOGY : 59. TAGUNG VOM 08. - 10.03.2005 IN STUTTGART-HOHENHEIM, FRANKFURT AM MAIN : DLG-VERL, DE, vol. 14, 8 March 2005 (2005-03-08), page 94, XP008132474, ISBN: 978-3-7690-4098-2

### SEQUENCE LISTING

<110> FBN - Leibniz-Institut für Nutztierbiologie, Dummerstorf
<120> Na+/Mg2+ exchanger
<130> PVA-24 699 EP
<160> 5
<170> PatentIn version 3.3
<210> 1
   <211> 513
   <212> PRT
   <213> Homo sapiens
<400> 1
   Met Ser Ser Lys Pro Glu Pro Lys Asp Val His Gln Leu Asn Gly Thr
   1               5                   10                  15
   Gly Pro Ser Ala Ser Pro Cys Ser Ser Asp Gly Pro Gly Arg Glu Pro
               20                  25                  30
   Leu Ala Gly Thr Ser Glu Phe Leu Gly Pro Asp Gly Ala Gly Val Glu
           35                  40                  45
   Val Val Ile Glu Ser Arg Ala Asn Ala Lys Gly Val Arg Glu Glu Asp
       50                  55                  60
   Ala Leu Leu Glu Asn Gly Ser Gln Ser Asn Glu Ser Asp Asp Val Ser
   65                  70                  75                  80
   Thr Asp Arg Gly Pro Ala Pro Pro Ser Pro Leu Lys Glu Thr Ser Phe
                   85                  90                  95
   Ser Ile Gly Leu Gln Val Leu Phe Pro Phe Leu Leu Ala Gly Phe Gly
               100                 105                 110
   Thr Val Ala Ala Gly Met Val Leu Asp Ile Val Gln His Trp Glu Val
           115                 120                 125
   Phe Gln Lys Val Thr Glu Val Phe Ile Leu Val Pro Ala Leu Leu Gly
       130                 135                 140
   Leu Lys Gly Asn Leu Glu Met Thr Leu Ala Ser Arg Leu Ser Thr Ala
   145                 150                 155                 160
   Ala Asn Ile Gly His Met Asp Thr Pro Lys Glu Leu Trp Arg Met Ile
                   165                 170                 175
   Thr Gly Asn Met Ala Leu Ile Gln Val Gln Ala Thr Val Val Gly Phe
               180                 185                 190
   Leu Ala Ser Ile Ala Ala Val Val Phe Gly Trp Ile Pro Asp Gly His
           195                 200                 205
   Phe Ser Ile Pro His Ala Phe Leu Leu Cys Ala Ser Ser Val Ala Thr
       210                 215                 220
   Ala Phe Ile Ala Ser Leu Val Leu Gly Met Ile Met Ile Gly Val Ile
   225                 230                 235                 240
   Ile Gly Ser Arg Lys Ile Gly Ile Asn Pro Asp Asn Val Ala Thr Pro
                   245                 250                 255
   Ile Ala Ala Ser Leu Gly Asp Leu Ile Thr Leu Ala Leu Leu Ser Gly
               260                 265                 270
   Ile Ser Trp Gly Leu Tyr Leu Glu Leu Asn His Trp Arg Tyr Ile Tyr
           275                 280                 285
   Pro Leu Val Cys Ala Phe Phe Val Ala Leu Leu Pro Val Trp Val Val
       290                 295                 300
   Leu Ala Arg Arg Ser Pro Ala Thr Arg Glu Val Leu Tyr Ser Gly Trp
   305                 310                 315                 320
   Glu Pro Val Ile Ile Ala Met Ala Ile Ser Ser Val Gly Gly Leu Ile
                   325                 330                 335
   Leu Asp Lys Thr Val Ser Asp Pro Asn Phe Ala Gly Met Ala Val Phe
               340                 345                 350
   Thr Pro Val Ile Asn Gly Val Gly Gly Asn Leu Val Ala Val Gln Ala
           355                 360                 365
   Ser Arg Ile Ser Thr Phe Leu His Met Asn Gly Met Pro Gly Glu Asn
       370                 375                 380
   Ser Glu Gln Ala Pro Arg Arg Cys Pro Ser Pro Cys Thr Thr Phe Phe
   385                 390                 395                 400
   Ser Pro Asp Val Asn Ser Arg Ser Ala Arg Val Leu Phe Leu Leu Val
                   405                 410                 415
   Val Pro Gly His Leu Val Phe Leu Tyr Thr Ile Ser Cys Met Gln Gly
   420 425 430
   Gly His Thr Thr Leu Thr Leu Ile Phe Ile Ile Phe Tyr Met Thr Ala
   435 440 445
   Ala Leu Leu Gln Val Leu Ile Leu Leu Tyr Ile Ala Asp Trp Met Val
   450 455 460
   His Trp Met Trp Gly Arg Gly Leu Asp Pro Asp Asn Phe Ser Ile Pro
   465 470 475 480
   Tyr Leu Thr Ala Leu Gly Asp Leu Leu Gly Thr Gly Leu Leu Ala Leu
   485 490 495
   Ser Phe His Val Leu Trp Leu Ile Gly Asp Arg Asp Thr Asp Val Gly
   500 505 510
   Asp
<210> 2
   <211> 1542
   <212> RNA
   <213> homo sapiens
<400> 2
   auguccucua agccagagcc gaaggacguc caccaacuga acgggacugg cccuucugcc 60
   ucucccugcu cuucagaugg cccagggaga gagcccuugg cugggaccuc agaguuccug 120
   gggccugaug gggcuggggu agagguggug auugagucuc gggccaacgc caaggggguu 180
   cgggaggagg acgcccugcu ggagaacggg agccagagca acgaaaguga cgacgucagc 240
   acagaccgug gcccugcgcc accuuccccg cucaaggaga ccuccuuuuc caucgggcug 300
   caaguacugu uuccauuccu ccuggcaggc uuugggaccg uggcugcugg caugguguug 360
   gacaucgugc agcacuggga agucuuccag aaggugacag aggucuucau ccuagugccu 420
   gcgcugcugg ggcucaaagg gaaccuggaa augacccugg caucaaggcu uuccacugca 480
   gccaacauug gacacaugga cacacccaag gagcucuggc ggaugaucac ugggaacaug 540
   gcccucaucc aggugcaggc cacgguggug ggcuuccugg cguccaucgc agccgucguc 600
   uuuggcugga ucccugaugg ccacuucagu auuccgcacg ccuuccugcu cugugcuagc 660
   agcguggcca cagccuucau ugccucccug guacugggua ugaucaugau uggagucauc 720
   auuggcucuc gcaagauugg gaucaaccca gacaacgugg ccacacccau ugcugccagc 780
   cugggcgacc ucaucaccuu ggcgcugcuc ucaggcauca gcuggggacu cuaccuggaa 840
   cugaaucacu ggcgauacau cuacccacug gugugugcuu ucuuuguggc ccugcugccu 900
   gucugggugg ugcuggcccg acgaagucca gccacaaggg agguguugua cucgggcugg 960
   gagccuguua ucauugccau ggccaucagc agugugggag gccucaucuu ggacaagacu 1020
   gucucagacc ccaacuuugc ugggauggcu gucuucacgc cugugauuaa ugguguuggg 1080
   ggcaaucugg uggcagugca ggccagccgc aucuccaccu uccugcacau gaauggaaug 1140
   cccggagaga acucugagca agcuccucgc cgcuguccca guccuuguac caccuucuuc 1200
   agcccugaug ugaauucucg cucagcccgg guccucuucc uccucguggu cccaggacac 1260
   cugguguucc ucuacaccau cagcuguaug cagggcgggc acaccacccu cacacucauc 1320
   uucaucaucu ucuauaugac agcugcacug cuccaggugc ugauucuccu guacaucgca 1380
   gacuggaugg ugcacuggau guggggccgg ggccuggacc cggacaacuu cuccauccca 1440
   uacuugacug cucuggggga ccugcuuggc acugggcucc uagcacucag cuuccauguu 1500
   cucuggcuca uaggggaccg agacacggau gucggggacu ag 1542
<210> 3
   <211> 1542
   <212> DNA
   <213> homo sapiens
<400> 3
   atgtcctcta agccagagcc gaaggacgtc caccaactga acgggactgg cccttctgcc      60
   tctccctgct cttcagatgg cccagggaga gagcccttgg ctgggacctc agagttcctg     120
   gggcctgatg gggctggggt agaggtggtg attgagtctc gggccaacgc caagggggtt    180
   cgggaggagg acgccctgct ggagaacggg agccagagca acgaaagtga cgacgtcagc     240
   acagaccgtg gccctgcgcc accttccccg ctcaaggaga cctccttttc catcgggctg     300
   caagtactgt ttccattcct cctggcaggc tttgggaccg tggctgctgg catggtgttg     360
   gacatcgtgc agcactggga agtcttccag aaggtgacag aggtcttcat cctagtgcct     420
   gcgctgctgg ggctcaaagg gaacctggaa atgaccctgg catcaaggct ttccactgca     480
   gccaacattg gacacatgga cacacccaag gagctctggc ggatgatcac tgggaacatg     540
   gccctcatcc aggtgcaggc cacggtggtg ggcttcctgg cgtccatcgc agccgtcgtc     600
   tttggctgga tccctgatgg ccacttcagt attccgcacg ccttcctgct ctgtgctagc     660
   agcgtggcca cagccttcat tgcctccctg gtactgggta tgatcatgat tggagtcatc     720
   attggctctc gcaagattgg gatcaaccca gacaacgtgg ccacacccat tgctgccagc     780
   ctgggcgacc tcatcacctt ggcgctgctc tcaggcatca gctggggact ctacctggaa     840
   ctgaatcact ggcgatacat ctacccactg gtgtgtgctt tctttgtggc cctgctgcct     900
   gtctgggtgg tgctggcccg acgaagtcca gccacaaggg aggtgttgta ctcgggctgg     960
   gagcctgtta tcattgccat ggccatcagc agtgtgggag gcctcatctt ggacaagact    1020
   gtctcagacc ccaactttgc tgggatggct gtcttcacgc ctgtgattaa tggtgttggg    1080
   ggcaatctgg tggcagtgca ggccagccgc atctccacct tcctgcacat gaatggaatg    1140
   cccggagaga actctgagca agctcctcgc cgctgtccca gtccttgtac caccttcttc    1200
   agccctgatg tgaattctcg ctcagcccgg gtcctcttcc tcctcgtggt cccaggacac    1260
   ctggtgttcc tctacaccat cagctgtatg cagggcgggc acaccaccct cacactcatc    1320
   ttcatcatct tctatatgac agctgcactg ctccaggtgc tgattctcct gtacatcgca    1380
   gactggatgg tgcactggat gtggggccgg ggcctggacc cggacaactt ctccatccca    1440
   tacttgactg ctctggggga cctgcttggc actgggctcc tagcactcag cttccatgtt    1500
   ctctggctca taggggaccg agacacggat gtcggggact ag                       1542
<210> 4
   <211> 1539
   <212> DNA
   <213> Mus musculus
<400> 4
   atgtcctcta agccagagcc aaaggacatc caccagccga atggtactgg ccctactcct      60
   tctccctgtt cttcggatgg ccctgggcga gagcccttgg ctgggacctc agagttcctg     120
   gggcctgatg gggtggaggt ggtagtgatt gagtcccgag ctaacgccaa gggaattcgg     180
   gaggaggacg ccctactgga gaacgggagc cagagtaacg agagtgacga tgtcagcaca     240
   gaccggggcc ccgcgccacc ctctccgctc aaggagacct ccttttccat tggactgcaa     300
   gtgctgttcc ccttcctact ggcaggcttt gggactgtgg cagctggcat ggtgctggac     360
   attgtgcagc actgggaggt cttccagaag gtgacagagg tcttcatcct ggtgcctgca     420
   ctgctggggc tcaaagggaa cttggaaatg accctggcgt cgaggctgtc cactgcagcc     480
   aacattggac aaatggacac acctaaggag ctctggcgga tgatcacggg gaatatggcc     540
   cttatccagg tacaagccac ggtggtaggc ttcctggcat ccatcgcagc cgtcgtcttt     600
   ggctggatcc ctgatggcca ctttagcatc ccacacgcct tcctgctatg cgccagcagt     660
   gtggctacag ccttcattgc ctccttggtc ctgggtatga tcatgatcgg agtcatcatt     720
   gggtctcgaa agattgggat caacccagac aacgtagcca cgcccatcgc cgctagcctg     780
   ggcgacctca tcaccttggc actcctctca ggcatcagct ggggactcta cctggaactg     840
   aagcactggc gatacatcta tcccctggtg tgtgccttct ttgtggccct gctgcctgtc     900
   tgggtggtgc tggcccgaag aagcccggcc acaagggagg tgttgtattc aggctgggag      960
   cctgtcatca ttgctatggc tatcagcagt gtgggaggtc tcatcctgga caagaccgtc    1020
   tcggacccca actttgcagg aatggctgtc ttcacacctg tgattaatgg tgtcgggggc    1080
   aatctggtgg ctgtgcaggc cagccgcatc tccaccttcc tccacatgaa cggcatgcca    1140
   ggggagaact ctgagccaac tcctcgccgc tgccccagtc catgcaccac cttcttcagc    1200
   cctgatgtga attcacgctc ggcccgtgtc ctcttcctcc ttgtggtccc aggacaccta    1260
   gtgttcctct acaccatcag ctgtatgcag ggcgggcaca ccaccctcac cctcatcttc    1320
   atcatcttct acatgactgc tgcactgctc caggtgctga ttctcctgta catcgcagac    1380
   tggatggtgc actggatgtg gggccggggc ctggaccccg acaacttctc catcccatac     1440
   ttgactgctt tgggggacct gcttggcacc ggactcctgg cactcagctt ccatgtgctc     1500
   tggctcattg gggacagaga cacagatgtt ggggactag                            1539
<210> 5
   <211> 512
   <212> PRT
   <213> Mus musculus
<400> 5
   Met Ser Ser Lys Pro Glu Pro Lys Asp Ile His Gln Pro Asn Gly Thr
   1               5                   10                  15
   Gly Pro Thr Pro Ser Pro Cys Ser Ser Asp Gly Pro Gly Arg Glu Pro
               20                  25                  30
   Leu Ala Gly Thr Ser Glu Phe Leu Gly Pro Asp Gly Val Glu Val Val
           35                  40                  45
   Val Ile Glu Ser Arg Ala Asn Ala Lys Gly Ile Arg Glu Glu Asp Ala
       50                  55                  60
   Leu Leu Glu Asn Gly Ser Gln Ser Asn Glu Ser Asp Asp Val Ser Thr
   65                  70                  75                  80
   Asp Arg Gly Pro Ala Pro Pro Ser Pro Leu Lys Glu Thr Ser Phe Ser
                   85                  90                  95
   Ile Gly Leu Gln Val Leu Phe Pro Phe Leu Leu Ala Gly Phe Gly Thr
               100                 105                 110
   Val Ala Ala Gly Met Val Leu Asp Ile Val Gln His Trp Glu Val Phe
           115                 120                 125
   Gln Lys Val Thr Glu Val Phe Ile Leu Val Pro Ala Leu Leu Gly Leu
       130                 135                 140
   Lys Gly Asn Leu Glu Met Thr Leu Ala Ser Arg Leu Ser Thr Ala Ala
   145 150 155 160
   Asn Ile Gly Gln Met Asp Thr Pro Lys Glu Leu Trp Arg Met Ile Thr
   165 170 175
   Gly Asn Met Ala Leu Ile Gln Val Gln Ala Thr Val Val Gly Phe Leu
   180 185 190
   Ala Ser Ile Ala Ala Val Val Phe Gly Trp Ile Pro Asp Gly His Phe
   195 200 205
   Ser Ile Pro His Ala Phe Leu Leu Cys Ala Ser Ser Val Ala Thr Ala
   210 215 220
   Phe Ile Ala Ser Leu Val Leu Gly Met Ile Met Ile Gly Val Ile Ile
   225 230 235 240
   Gly Ser Arg Lys Ile Gly Ile Asn Pro Asp Asn Val Ala Thr Pro Ile
   245 250 255
   Ala Ala Ser Leu Gly Asp Leu Ile Thr Leu Ala Leu Leu Ser Gly Ile
   260 265 270
   Ser Trp Gly Leu Tyr Leu Glu Leu Lys His Trp Arg Tyr Ile Tyr Pro
   275 280 285
   Leu Val Cys Ala Phe Phe Val Ala Leu Leu Pro Val Trp Val Val Leu
   290 295 300
   Ala Arg Arg Ser Pro Ala Thr Arg Glu Val Leu Tyr Ser Gly Trp Glu
   305 310 315 320
   Pro Val Ile Ile Ala Met Ala Ile Ser Ser Val Gly Gly Leu Ile Leu
   325 330 335
   Asp Lys Thr Val Ser Asp Pro Asn Phe Ala Gly Met Ala Val Phe Thr
   340 345 350
   Pro Val Ile Asn Gly Val Gly Gly Asn Leu Val Ala Val Gln Ala Ser
   355 360 365
   Arg Ile Ser Thr Phe Leu His Met Asn Gly Met Pro Gly Glu Asn Ser
   370 375 380
   Glu Pro Thr Pro Arg Arg Cys Pro Ser Pro Cys Thr Thr Phe Phe Ser
   385 390 395 400
   Pro Asp Val Asn Ser Arg Ser Ala Arg Val Leu Phe Leu Leu Val Val
                   405                 410                 415
   Pro Gly His Leu Val Phe Leu Tyr Thr Ile Ser Cys Met Gln Gly Gly
               420                 425                 430
   His Thr Thr Leu Thr Leu Ile Phe Ile Ile Phe Tyr Met Thr Ala Ala
           435                 440                 445
   Leu Leu Gln Val Leu Ile Leu Leu Tyr Ile Ala Asp Trp Met Val His
       450                 455                 460
   Trp Met Trp Gly Arg Gly Leu Asp Pro Asp Asn Phe Ser Ile Pro Tyr
   465                 470                 475                 480
   Leu Thr Ala Leu Gly Asp Leu Leu Gly Thr Gly Leu Leu Ala Leu Ser
                   485                 490                 495
   Phe His Val Leu Trp Leu Ile Gly Asp Arg Asp Thr Asp Val Gly Asp
               500                 505                 510

## Description

The magnesium ion Mg²⁺ is a vital element essentially involved in numerous physiological processes at the cell, organ and organism level [Kolisek et al., 2007]. Therefore, the biologically active free intracellular Mg²⁺ concentration ([Mg²⁺]ᵢ) has to be adjusted to its optimal level of 0.5 to 1.2 mM. In this process transmembrane extrusion of Mg²⁺ has been shown to be critically involved (Schweigel et al., 2006). So far, there is no evidence for the existence of an outwardly oriented Mg-ATPase, however, by functional studies two main efflux systems, namely an anion-dependent mechanism [Ödblom and Handy, 1999] and a putative Na⁺/Mg²⁺ exchanger [Schweigel et al., 2006, 2009], have been proposed. After its first description in chicken and turkey red blood cells (Günther et al., 1984), Na⁺-dependent efflux of intracellular Mg²⁺ has been shown to operate in the majority of examined cells including ventricular myocytes, vascular smooth muscle cells, hepatocytes, thymocytes, neurons, renal and gastrointestinal epithelial cells (Kolisek et al., 2007; Schweigel et al., 2006). The Na⁺/Mg²⁺ exchanger is also a candidate mechanism for the extrusion of Mg²⁺ across the basolateral membrane of the Mg²⁺ absorbing and reabsorbing intestinal and renal epithelia (Quamme, 1989) making it important for control of body Mg²⁺ homeostasis (balance). The near-ubiquity of the Na⁺/Mg²⁺ exchanger attests to its functional importance, as is substantiated by its participation in the pathogenesis of a growing collection of human diseases (Kimura, 2007). Disturbance or dysregulation of the antiporter seem to be involved in primary hypertension, ischemic heart disease, eclampsia, diabetes and cystic fibrosis to name a few (Kisters et al., 1999; Vormann et al., 1992; Kimura, 2007; Kumeda and Inaba, 2007).

All approaches used so far, in order to identify the encoding gene(s) and/or to solve the protein structure of the Na⁺/Mg²⁺ exchanger, e.g. establishment of a high (> 100 mM)-Mg²⁺ resistant cell line (Watanabe et al., 2005), failed. Only the protein structure of the Na⁺/Mg²⁺ exchanger will allow for a detailed investigation of the modalities of its operation and regulation as well as for the development of specific inhibitors or agonists.

Within the last years several candidate genes have been characterized encoding for proteins involved in the regulation of eukaryotic cells Mg²⁺ homeostasis (summarized in [Sponder et al., 2010]). Only few of them have been clearly shown to be directly and specifically involved in Mg²⁺ transport processes. The best characterized are the TRPM6 and TRPM7 channels of the Melastatin-related Transient Receptor Potential family considered as being the major Mg²⁺ influx pathways of mammalian cells and the mitochondrial channel MRS2 (Mitochondrial RNA Splicing member 2) that mediates Mg²⁺ influx into the mitochondrial matrix [Schmitz, 2003; Kolisek, 2003, Schlingmann et al., 2002]. Other putative Mg²⁺ transporters, found by a differential gene expression approach (Goytain and Quamme, 2005a-d, Goytain et al., 2007), are MagT1 (Magnesium Transporter 1), ACDP 2 (Ancient Conserved Domain Protein; subtype 2), the protein NIPA1 (Nonimprinted in Prader-Willi/Angelman), SLC41A1 and SLC41A2 (Solute Carrier family 41; subfamily A; members 1 and 2).

WO 0190360 discloses human SLC41A1.

When over-expressed in *Xenopus laevis* oocytes, a genetically heterologous system, the group of Quamme [Goytain and Quamme, 2005a-d; Goytain et al., 2007] characterized mouse (m)SLC41A1, mSLC41A2, mACDP 2 and mMagT1 all as being Mg²⁺ channels mediating influx of the ion. However, with the exception of MagT1 the transport was not very specific to Mg²⁺ and, when over-expressed in human-derived cells hSLC41A2 [Sahni et al., 2007], hMagT1 [Zhou and Clapham, 2009] and hSLC41A1 (Kolisek et al., 2008) all failed to induce Mg²⁺ evoked currents. Moreover, by using tet-induced over-expression of hSLC41A1 in HEK293 cells strong evidence was found for human SLC41A1 as being a Mg²⁺ carrier localized to the cytoplasmatic membrane and mediating efflux of the ion [Kolisek et al., 2008]. *SLC41A1* has been shown to have partial sequence homology [Wabakken et al., 2003] to a specific region in the transmembrane domain of the bacterial Mg²⁺ transport system MgtE described by Smith et al. (1995). It is interesting to note, that the MgtE proteins have been discovered by complementation assays and that they show no homology to other groups of bacterial Mg²⁺ transporters operating as pumps (MgtA, MgtB) or channels (CorA). Human [Wabbaken et al., 2003] and mice [Goytain and Quamme, 2005c] *SLC41A1* transcripts have been found to be highly abundant in heart, brain, kidney, liver, muscle, testis, thyroid gland, lymphocytes and to a lesser extent in most other tissues including colon and small intestine. Neither the functions of MgtE nor of SLC41A1 have been thoroughly characterized.

Thus, the object of the present invention was the identification of the encoding gene(s) and the salvation of the protein structure of the Na⁺/Mg²⁺ exchanger.

The object was solved by the finding that the protein SLC41A1 is the cellular Na⁺/Mg²⁺ exchanger. It could be proven that the protein SLC41A1 mediates Mg²⁺ extrusion and its mode of operation could be clarified. In the following, the naming SEQ ID NO 1 is used for the protein SLC41A1 (GenBank Accession No. NP_776253) and the naming SEQ ID NO 2 is used for the underlying mRNA sequence of SLC41A1 (GenBank Accession No. NM_173854). The cDNA sequence is represented in SEQ ID NO 3. SEQ ID NO 4 represents the SLC41A1 from mouse (Mus musculus, complete cds, GenBank Accession No. BC116280.1, CDS: 802>2340) and SEQ I D NO 5 is the related protein sequence (Mus musculus, NCBI Reference Sequence: NP_776290.1).

The invention relates to methods to identify new SLC41A1 functions at the cell, tissue, organ and organism level. In part, it is related to methods useful in (a) identifying molecules that bind SLC41A1 polypeptides, which (b) modulate SLC41A1 related Na⁺/Mg²⁺ exchanger activity or its cellular or tissue specific expression.

Thus, the invention comprises SLC41A1 mutation libraries. Also disclosed are SLC41A1 specific antibodies and their generation as well as an inducible conditional knock out mice model and a conditional inducible knock out cell line.

The SLC41A1 mutation libraries comprise at least one of the following mutation clusters i, ii, iii, iv of the amino acid sequence of SEQ ID NO 1 (indicated in brackets are the triplet changes of the cDNA of SEQ ID NO 3):
i) One mutation library comprises site-directed mutants of SEQ ID NO 1, wherein a single amino acid (aa) is substituted in position: 21 S, 25S, 73S, 76S, 80S, 89S, 97S, 158S, 309S, 394S, 408S, 81T, 168T, 509T and 278Y, respectively.
   In other words, a single aa serine (S), threonine (T) or tyrosine (Y) is replaced at one of the following positions of SEQ ID NO 1 by another aa:
   21 S>N (TCT>AAT), 25 S>Q (TCA>CAA), 73 S>N (AGC>AAC), 76 S>N (AGT>AAT), 80 S>N (AGC>AAC), 89 S>N (TCC>AAC), 97 S>N (TCC>AAC), 158 S>N (TCC>AAC), 309 S>N (AGT>AAT), 394 S>N (AGT>AAT) and 408 S>Q (TCA>CAA), 81 T>Q (ACA>CAA), 168 T>Q (ACA>CAA), 509 T>Q (ACG>CAG) and 278 Y>N (TAC>AAC), respectively. Comprised are also combinations of these mutations, i.e. at least one mutation of SEQ ID NO 1, wherein a plurality (2-15) of these substitutions is present. Preferably, 2-5 aa's are replaced, most preferably 2 or 3. In other words, the mutation library of cluster i) consists of 15 peptides with single aa mutation and at least one mutation of SEQ ID NO 1, wherein a plurality (2-15) of these substitutions is present.
ii) Another mutation library comprises mutations of the transmembrane domains (TM) and N-, C- termini mutants of SEQ ID NO 1:
   Based on SEQ ID NO 1, respective deletions (Δ) of the amino acid sequences were present in:
   a) N-terminus and C-terminus flanking region: Δaa 2-30, Δaa 31-60, Δaa 61-90 and Δaa 2-60, Δaa 2-90, Δaa 504-513, respectively (6 mutated proteins) and
   b) membrane spanning domains of SEQ ID NO 1 of highly probable TM10 model (TMP): Δaa 96-205 (TM1+TM2); Δaa 225-279 (TM3+TM4); Δaa 286-337(TM5+TM6); Δaa 348-427 (TM7+TM8) and Δaa 439-503 (TM9+TM10), respectively (5 mutated proteins)
      and
   c) membrane spanning domains of less-probable TM11 model (SOSUI): Δaa 99-150 (TM1+TM2); Δaa 180-241 (TM3+TM4); Δaa 256-306 (TM5+TM6); Δaa 317-368 (TM7+TM8); Δaa 408-459 (TM9+TM10) and Δaa 437-503 (TM10+TM11), respectively (6 mutated proteins).
      Overall, cluster ii) comprises 17 mutated proteins.
iii) Another mutation library comprises leucine zipper (LZ) mutants of SEQ ID NO 1:
   These SLC41A1 mutants comprise a deletion (Δ) respective to SEQ ID NO 1 of
   a) Δaa 482-503 and
   b) aa substitutions in LZ domain in positions 482 L>V (TTG>GTG), 489 L>V (CTT>GTT), 494 L>V (CTA>GTA), 496 L>V (CTC>GTC), 501 L>V (CTC>GTC) and 503 L>V (CTC>GTC), respectively (7 mutated proteins).
iv) Another mutation library comprises external loops (EL) mutants of SEQ ID NO 1:
   These SLC41A1 mutants comprise deletions (Δ) respective to SEQ ID NO 1 of the amino acid sequences in EL: Δaa 128-179, Δaa 247-254, Δaa 310-315, Δaa 369-405 and Δaa 468-478 respectively (5 mutated proteins).

The mutations described in cluster i) are believed to be useful for the identification of SLC41A1 variants showing a modified phosphorylation pattern and therefore, an impaired or modified function of the carrier.

Mutations in the membrane spanning domains (cluster ii) and EL mutations (cluster iv) are believed to be useful for the identification of the minimal sequential configuration necessary to maintain the Mg²⁺/Na⁺ exchange (efflux) function of SLC41A1 in vivo and of the Mg²⁺ binding motif(s) / the Mg²⁺ binding site. Thus, they will be helpful to generate more active SLC41A1 variants or SLC41A1 variants with impaired function of the SLC41A1 carrier.

The LZ domain is known to be critical in dimerization and thus, for the complex forming ability of SLC41A1 which is essential for its normal function as a carrier (Kolisek et al., 2008). Mutations in the LZ domain (cluster iii) are believed to be helpful in the generation of SLC41A1 variants with changed transport and regulatory characteristics and/or mode of action.

Each of these sequences is combined in a mutation library, which comprises at least all sequences of one of the mutation clusters i) to iv). The most preferred embodiment is a mutation library, which combines all four mutation clusters i) to iv).

The expected outcome of mutation clusters i, ii and iv is also a definitive confirmation of the SLC41A1 orientation in cytoplasmatic membrane of the cell. As shown by Kolisek et al. (2008), a predicted model which comprises 10 TMs and both N- and C- terminus oriented intracellularly is most likely.

Respective mutated SLC41A1 inserts (from cluster i,ii,iii and/or iv) are cloned into cloning vector *pUC19,* the selected clones are controlled for the proper insert orientation and sequenced for verification purposes. The constructed library is subcloned into respective mammalian expression vector(s) and used for functional screening in an inducible conditional SLC41A1 knockout cell line or into cells of a transgenic conditional knockout animal, which both will be described in more detail below.

Also disclosed herein is the use of a protein consisting of an amino acid sequence SEQ ID NO 1 or a protein fragment thereof, in which one or more amino acids are substituted, deleted or added and which has an identity on the amino acid level with SEQ ID NO 1 of at least 90%, preferably at least 95%, more preferably 98%, for the generation and identification of specific antibodies against the cellular Na⁺/Mg²⁺ exchanger.

Below, the term "identity of at least 90%" always means that this comprises preferably an identity of at least 95%, more preferably an identity of 98%, regardless whether proteins or DNAs are concerned.

The related method of identifying antibodies against the cellular Na⁺/Mg²⁺ exchanger comprises the steps of:
- contacting a protein having SEQ ID NO 1 or a protein fragment thereof, in which one or more amino acids are substituted, deleted or added and which has an identity on the amino acid level with SEQ ID NO 1 of at least 90% with a primary antibody under conditions that allow complex formation of the primary antibody with the protein or the protein fragment and
- detecting the complex.

The complex is preferably detected by a secondary antibody. In a preferred embodiment, the primary und/or the secondary antibody comprise(s) a label, which is selected from the group of radioactive label, fluorescent label, enzymatic label, or biotin label.

Also disclosed herein is a kit for identifying antibodies against the Na⁺/Mg²⁺ exchanger, wherein the kit comprises a protein consisting of an amino acid sequence of SEQ ID NO 1 or a protein fragment thereof, in which one or more amino acids are substituted, deleted or added and which has an identity on the amino acid level with SEQ ID NO 1 of at least 90%.

Also disclosed herein is the use of a protein consisting of an amino acid sequence of SEQ ID NO 1 or a protein fragment thereof, in which one or more amino acids are substituted, deleted or added and which has an identity on the amino acid level with SEQ ID NO 1 of at least 90% in a method which uses the protein in order to generate and produce antibodies, preferably monoclonal antibodies, against the Na⁺/Mg²⁺ exchanger.

Several antibodies are disclosed which selectively bind to the Na⁺/Mg²⁺ echanger. Specifically seven antibodies, wherein each specifically recognises one of the following epitopes
ESRANAKGVREEDALLENGS,
ESDDVSTDRGPAPPSPLKE,
LKGNLEMTLASRLSTAAN,
STAANIGHMDTPKEL,
SSVGGLILDKTVSDPN,
PGENSEQAPRRCPSPCTT or
ADWMVHWMWGRGLDPDN of SEQ ID NO 1.

These antibodies can be used for the detection of the presence, absence and or amount and for the interaction with the cellular Na⁺/Mg²⁺ exchanger, which has a protein having the amino acid sequence of SEQ ID NO 1 or a protein fragment thereof, in which one or more amino acids are substituted, deleted or added and which has an identity on the amino acid level with SEQ ID NO 1 of at least 90%. The antibodies may be polyclonal or monoclonal but monoclonal antibodies are preferred.

Also disclosed herein is the use of a protein consisting of an amino acid sequence of SEQ ID NO 1 or a protein fragment thereof, in which one or more amino acids are substituted, deleted or added and which has an identity on the amino acid level with SEQ ID NO 1 of at least 90% for the identification of specific protein binding partners that can form stable or transient protein complexes with the cellular Na⁺/Mg²⁺ exchanger.

The related method of identifying specific protein complex partners for the Na⁺/Mg²⁺ exchanger, comprises the steps of:
- contacting a cell, which contains a protein of amino acid sequence SEQ ID NO 1 or a fragment thereof, in which one or more amino acids are substituted, deleted or added and which has an identity on the amino acid level with SEQ ID NO 1 of at least 90%
   with a candidate substance under conditions that allow for an interaction of the candidate substance with the protein or the fragment,
- determining the the free or total intracellular Mg²⁺ concentration or the extracellular Mg²⁺ concentration, and
- comparing the Mg²⁺ concentration as determined with the free or total intracellular Mg²⁺ concentration or the extracellular Mg²⁺ concentration in the absence of the candidate substance.

Also disclosed herein is a kit for identifying specific protein binding partners forming stable or transient protein complexes with the Na⁺/Mg²⁺ exchanger, wherein the kit comprises a protein having an amino acid sequence of SEQ ID NO 1 or a protein fragment thereof, in which one or more amino acids are substituted, deleted or added and which has an identity on the amino acid level with SEQ ID NO 1 of at least 90%.

Especially the substances listed below prove suitable as protein complex partner for the Na⁺/Mg²⁺ exchanger, which is identical to a protein having an amino acid sequence of SEQ ID NO 1 or a protein fragment thereof, in which one or more amino acids are substituted, deleted or added and which has an identity on the amino acid level with SEQ ID NO 1 of at least 90%:
3-beta-hydroxysteroid-delta(8),delta(7)-isomerase (OS=homo sapiens, UniProt ID Q15125); 3-beta-hydroxysteroid-delta(8),Delta(7)-isomerase OS=Rattus norvegicus, UniProt ID Q9JJ46); ATP synthase subunit a (OS=Cricetulus griseus, UniProt ID P14413); ATP synthase subunit a (OS=homo sapiens, UniProt ID P00846); ATP synthase subunit a (OS=Pan troglodytes, UniProt ID Q9T9W0); ATP synthase subunit a (OS=Tachyglossus aculeatus aculeatus, UniProt ID Q8W9G8); B-cell receptor-associated protein 31 (OS=Homo sapiens, UniProt ID P51572); Immediate early response 3-Interaeting protein 1 (OS=Homo sapiens, UniProt ID Q9Y5U9); Keratinocyte-associated protein 2 (OS=Homo sapiens, UniProt ID Q8N6L1); Kunitz-type protease inhibitor 2 (OS=Homo sapiens, UniProt ID 043291); Peptidyl-prolyl cis-trans isomerase B (OS=Homo sapiens, UniProt ID P23284); Probable ergosterol biosynthetic protein 28 (OS=Pongo abelii, UniProt ID Q5R589); Protein disulfide-isomerase A6 (OS=Homo sapiens, UniProt ID Q15084); Protein YIPF6 (OS=Homo sapiens, UniProt ID Q96EC8); Putative uncharacterized protein CXorf62 (OS=Homo sapiens, UniProt ID Q8N2A0); Signal peptidase complex subunit 1 (OS=Pongo abelii, UniProt ID Q5RF96); Stress-associated endoplasmic reticulum protein 1 (OS=Rattus norvegicus, UniProt ID Q9R2C1); Translocon-associated protein subunit gamma (OS=Homo sapiens, UniProt ID Q9UNL2); Transmembrane protein 147 (OS=Homo sapiens, UniProt ID Q9BVK8); Transmembrane protein 14A (OS=Homo sapiens, UniProt ID Q9Y6G1); Uncharacterized protein C10orf35 (OS=Homo sapiens, UniProt ID Q96D05); Uncharacterized protein C4orf3 (OS=Homo sapiens, UniProt ID Q8WVX3); UPF0414 transmembrane protein C20orf30 (OS=Homo sapiens, UniProt ID Q96A57); UPF0480 protein C15orf24 homolog (OS=Macaca fascicularis, UniProt ID Q4R5V2); and UPF0480 protein C15orf24 (OS=Homo sapiens, UniProt ID Q9NPA0).

Preferred molecules out of these are 3-beta-hydroxysteroid-delta(8),delta(7)-isomerase (OS=Homo sapiens, UniProt ID Q15125); B-cell receptor-associated protein 31 (OS=Homo sapiens, UniProt ID P51572); Peptidyl-prolyl cis-trans isomerase (OS=Homo sapiens, UniProt ID P23284) and UPF0480 protein C15orf24 (OS=Macaca fascicularis, UniProt ID Q4R5V2).

These SLC41A1 complex-forming partners were screened out by the split-ubiquitin method, which is well known in the art and are listed in detail in Table 1. The ubiquitin-based split protein sensor (USPS) is the method that makes it possible to detect and monitor protein-protein interactions as a function of time, at the natural sites of these interactions in a living cell. Details on the method can be found in Johnsson and Varshavsky, 1994.

The more often the screened molecules occur in the list given in Table 1, the higher is their ability of being a binding partner for SLC41A1, i.e. the more clones revealed interaction with SLC41A1. In order to shorten the table, multiple entries were deleted and only the frequency of occurrence (Occurrence) is indicated as a number. The singletons listed in Table 2 are the weakest partners. They are organized from the strongest to the weakest.

**Table 1**

| **protein name (description)** | **uniprot ID** | **occurrence** |
|---|---|---|
| 3-beta-hydroxysteroid-Delta(8),Delta(7)-isomerase OS=Homo sapiens GN=EBP PE=1 SV=3 | Q15125 | 24 |
| 3-beta-hydroxysteroid-Delta(8),Delta(7)-isomerase OS=Rattus norvegicus GN=Ebp PE=2 SV=3 | Q9JJ46 | 1 |
| ATP synthase subunit a OS=Cricetulus griseus GN=MT-ATP6 PE=3 SV=1 | P14413 | 1 |
| ATP synthase subunit a OS=Homo sapiens GN=MT-ATP6 PE=1 SV=1 | P00846 | 1 |
| ATP synthase subunit a OS=Pan troglodytes GN=MT-ATP6 PE=3 SV=1 | Q9T9W0 | 1 |
| ATP synthase subunit a OS=Tachyglossus aculeatus aculeatus GN=MT-ATP6 PE=3 SV=1 | Q8W9G8 | 1 |
| B-cell receptor-associated protein 31 OS=Homo sapiens GN=BCAP31 PE=1 SV=3 | P51572 | 10 |
| Immediate early response 3-interacting protein 1 OS=Homo sapiens GN=IER3IP1 PE=1 SV=1 | Q9Y5U9 | 3 |
| Keratinocyte-associated protein 2 OS=Homo sapiens GN=KRTCAP2 PE=1 SV=1 | Q8N6L1 | 2 |
| Kunitz-type protease inhibitor 2 OS=Homo sapiens GN=SPINT2 PE=1 SV=2 | 043291 | 2 |
| Peptidyl-prolyl cis-trans isomerase B OS=Homo sapiens GN=PPIB PE=1 SV=2 | P23284 | 5 |
| Probable ergosterol biosynthetic protein 28 OS=Pongo abelii PE=2 SV=1 | Q5R589 | 2 |
| Protein disulfide-isomerase A6 OS=Homo sapiens GN=PDIA6 PE=1 SV=1 | Q15084 | 11 |
| Protein YIPF6 OS=Homo sapiens GN=YIPF6 PE=2 SV=2 | Q96EC8 | 2 |
| Putative uncharacterized protein Cxorf62 OS=Homo sapiens GN=Cxorf62 PE=2 SV=1 | Q8N2A0 | 2 |
| Signal peptidase complex subunit 1 OS=Pongo abelii GN=SPCS1 PE=3 SV=1 | Q5RF96 | 2 |
| Stress-associated endoplasmic reticulum protein 1 OS=Rattus norvegicus GN=Serp1 PE=1 SV=2 | Q9R2C1 | 4 |
| Translocon-associated protein subunit gamma OS=Homo sapiens GN=SSR3 PE=1 SV=1 | Q9UNL2 | 4 |
| Transmembrane protein 147 OS=Homo sapiens GN=TMEM147 PE=1 SV=1 | Q9BVK8 | 2 |
| Transmembrane protein 14A OS=Homo sapiens GN=TMEM14A PE=2 SV=1 | Q9Y6G1 | 2 |
| Uncharacterized protein C10orf35 OS=Homo sapiens GN=C10orf35 PE=1 SV=1 | Q96D05 | 2 |
| Uncharacterized protein C4orf3 OS=Homo sapiens GN=C4orf3 PE=2 SV=1 | Q8WVX3 | 2 |
| UPF0414 transmembrane protein C20orf30 OS=Homo sapiens GN=C20orf30 PE=1 SV=1 | Q96A57 | 3 |
| UPF0480 protein C15orf24 homolog OS=Macaca fascicularis GN=QtsA-20627 PE=2 SV=1 | Q4R5V2 | 1 |
| UPF0480 protein C15orf24 OS=Homo sapiens GN=C15orf24 PE=1 SV=1 | Q9NPA0 | 4 |

Further Singletons are listed below in table 2.

**Table 2**

| Protein name (description) | UniProt ID |
|---|---|
| ADP-ribosylation factor-like protein 6-interacting protein 4 OS=Homo sapiens GN=ARL6IP4 PE=1 SV=1 | Q66PJ3 |
| Elongation of very long chain fatty acids protein 4 OS=Homo sapiens GN=ELOVL4 PE=2 SV=1 | Q9GZR5 |
| Proteolipid protein 2 OS=Homo sapiens GN=PLP2 PE=1 SV=1 | Q04941 |
| Putative P2Y purinoceptor 10 OS=Homo sapiens GN=P2RY10 PE=2 SV=1 | 000398 |
| Transmembrane protein 106C OS=Homo sapiens GN=TMEM106C PE=2 SV=1 | Q9BVX2 |
| Solute carrier family 35 member B1 OS=Homo sapiens GN=SLC35B1 PE=2 SV=1 | P78383 |
| Thioredoxin-related transmembrane protein 1 OS=Homo sapiens GN=TMX1 PE=1 SV=1 | Q9H3N1 |
| Transmembrane emp24 domain-containinq protein 10 OS=Homo sapiens GN=TMED10 PE=1 SV=2 | P49755 |
| Putative uncharacterized protein C3orf66 OS=Homo sapiens GN=C3orf66 PE=5 SV=1 | Q6ZWE1 |
| Probable phospholipid-transporting ATPase ID OS=Homo sapiens GN=ATP8B2 PE=1 SV=2 | P98198 |
| Signal recognition particle 9 kDa protein OS=Homo sapiens GN=SRP9 PE=1 SV=2 | P49458 |
| Zinc transporter ZIP13 OS=Rattus norvegicus GN=SIc39a13 PE=2 SV=1 | Q2M1K6 |
| Phosphatidate phosphatase PPAPDC1B OS=Mus musculus GN=Ppapdc1b PE=2 SV=1 | Q3UMZ3 |
| WW domain-binding protein 11 OS=Rattus norvegicus GN=Wbp11 PE=2 SV=1 | Q5PQQ2 |
| Emerin OS=Homo sapiens GN=EMD PE=1 SV=1 | P50402 |
| Transmembrane 6 superfamily member 1 OS=Homo sapiens GN=TM06F1 PE=2 SV=2 | Q9BZW5 |
| C-4 methylsterol oxidase OS=Pongo abelii GN=SC4MOL PE=2 SV=1 | Q5R574 |
| Single-stranded DNA-binding protein 2 OS=Homo sapiens GN=SSBP2 PE=1 SV=2 | P81877 |
| Putative Uncharacterized protein C14orf165 OS=Homo sapiens GN=C14orf165 PE=4 SV=1 | Q86U02 |
| Zinc finger protein 714 OS=Homo sapiens GN=ZNF714 PE=2 SV=1 | Q96N38 |
| Calcium release-activated calcium channel protein 1 OS=Homo sapiens GN=ORAI1 PE=1 SV=2 | Q96D31 |
| Transmembrane BAX inhibitor motif-containing protein 4 OS=Homo sapiens GN=TMBIM4 PE=1 SV=2 | Q9HC24 |
| NADH-ubiquinone oxidoreductase chain 3 OS=Homo sapiens GN=MT-ND3 PE=1 SV=1 | P03897 |
| ADP-ribosylation factor-like protein 6-interacting protein 6 OS=Homo sapiens GN=ARL6IP6 PE=1 SV=1 | Q8N065 |
| Ribonuclease kappa OS=Mus musculus GN=Rnasek PE=2 SV=1 | Q8K3C0 |
| Wiskott-Aldrich syndrome protein homolog OS=Mus musculus GN=Was PE=1 SV=1 | P70315 |
| Cytochrome c oxidase subunit 2 OS=Homo sapiens GN=MT-CO2 PE=1 SV=1 | P00403 |
| Melanoma-associated antigen D1 OS=Mus musculus GN=Maged1 PE=1 SV=1 | Q9QYH6 |
| Probable low affinity copper uptake protein 2 OS=Homo sapiens GN=SLC31A2 PE=2 SV=1 | 015432 |
| Protein cornichon homolog 4 OS=Pongo abelii GN=CNIH4 PE=2 SV=1 | Q5R9M4 |
| Mitochondrial carrier homolog 2 OS=Pongo abelii GN=MTCH2 PE=2 SV=2 | Q5R5M0 |
| Squalene synthetase OS=Pongo abelii GN=FDFT1 PE=2 SV=1 | Q5R6U3 |
| RING finger protein 114 OS=Pan troglodytes GN=RNF114 PE=2 SV=1 | Q6J212 |
| Interferon alpha-inducible protein 6 OS=Homo sapiens GN=IFI6 PE=2 SV=2 | P09912 |
| Protein tyrosine phosphatase receptor type C-associated protein OS=Homo sapiens GN=PTPRCAP PE=1 SV=1 | Q14761 |
| Protein FAM192A OS=Homo sapiens GN=FAM192A PE=1 SV=1 | Q9GZU8 |
| Intercellular adhesion molecule 2 OS=Homo sapiens GN=ICAM2 PE=1 SV=2 | P13598 |
| Leptin receptor gene-related protein OS=Homo sapiens GN=LEPROT PE=1 SV=1 | O15243 |

Also disclosed herein is the use of said molecules as protein complex partners for the Na⁺/Mg²⁺ exchanger thereby regulating or modifying its function. Additionally, these specific protein binding partners are intended for the use in therapeutical applications, especially for the treatment of the diseases mentioned below (Table 4).

In the above tables 1 and 2 underlined names indicate highly connected proteins, found in the majority of DUALmembrane SU-YTH screens. Highly connected proteins include chaperones, stress-induced endoplasmatic reticulum proteins, components of the translocon machinery and proteins involved in membrane protein sorting and transport.

The most prominent candidate binding partner is 3-beta-hydroxysteroid-Delta(8),Delta(7)-isomerase, an enzyme which catalyzes the conversion of Delta(8)-sterols to their corresponding Delta(7)-isomers. In the screening 24 clones were identified where 3-beta-hydroxysteroid-Delta(8),Delta(7)-isomerase interacted with SLC41A1. Defects in 3-beta-hydroxysteroid-Delta(8),Delta(7)-isomerase are the cause of chondrodysplasia punctata also known as Conradi-Hunermann-Happle syndrome (Derry et al, 1999). As for the other candidate binding partners identified, the nature of interaction between SLC41A1 and 3-beta-hydroxysteroid-Delta(8),Delta(7)-isomerase is unknown. Its identification will help to understand the link between disturbed cholesterol metabolism and magnesium insufficiency in mammals.

Ten clones revealed interaction between SLC41 and B-cell receptor associated-protein 31. It is expected to play a role in anterograde transport of membrane proteins from the endoplasmic reticulum to the Golgi. It may be also involved in CASP8-mediated apoptosis.

The third strongest SLC41A1-binding candidates were Peptidyl-prolyl cis-trans isomerase and UPF0480 protein C15orf24.

The rest of the identified SLC41A1 binding partners, found in more than only one clone, is merely represented by poorly characterized or uncharacterized transmembrane proteins, proteins involved in mediating the cellular response to mitogenic signal (immediate early-response 3-interacting protein; SABiosciences) and endoplasmatic reticulum-located probable ergosterol biosynthetic protein 28, transmembrane protein 147 and APH-1-similar core component of nicalin-nomo complexes. One interesting candidate is the Kunitz type protease inhibitor 2. When unfunctional, the protein cause a rare, inherited diarrhea of infancy named congenital secretory sodium diarrhea type 3 (DIAR3) or congenital sodium diarrhea (CSD).

Interesting binding partners among the singletons are: probable low affinity copper uptake protein 2, mitochondrial carrier homologue 2 (transporter of an unknown solute), calcium release-activated calcium channel protein 1, zinc transporter ZIP13 and solute carrier family 35 member B1 which is likely to be an endoplasmatic reticulum located sugar transporter (Uniprot).

Further substances, which either increase or decrease the SLC41A1-related Na⁺/Mg²⁺ exchanger activity and are thus called modulators, are given in Table 3. These substances modulate SLC41A1-related Na⁺/Mg²⁺ exchanger activity either by direct interaction with structural components of the SLC41A1 molecule or by the induction of cellular signal cascades resulting in modifications of the SLC41A1 protein. Therefore, some of these modulators are hormones or elements of cellular signal cascades. Main sites for posttranslational modifications and thus, regulation/modulation of SLC41A1-related Na⁺/Mg²⁺ exchanger activity were the following:
- Glycosylation side: amino acid residue N-475
- cAMP-dependent PK phosphorylation sides: S-157, S-308, S-393, T-508
- PK C phosphorylation sides: T-80, T-167, T-387, S-407
- Casein kinase II motif at residue S-317
- Myristoylation sequence at G-143

**Table 3**

| **Modulator** | **Chemical name** | **Molecular weight (g/mol)** | **Dosage (µM)** | **Effect on Na⁺/Mg²⁺ exchanger** | **Further Effect on** |
|---|---|---|---|---|---|
| Imipramine | 10,11-Dihydro-*N*,*N-*dimethyl-5*H*-dibenz-[*b*,*f*]azepin-5-propanamin | 280.4 | 250 | Inhibition | Na⁺ channel blocker |
| Quinidin | (2-ethenyl- 4-azabicyclo [2.2.2] oct- 5-yl)- (6-methoxyquinolin- 4-yl)-methanol | 324.4 | 100 | Inhibition | K⁺ channel blocker |
| DIDS | 4',4'-diisothiocyanatodihydrostil bene-',2'-disulfonic acid, disodium salt | 500.48 | 100 | Inhibition | Cl⁻ channel inhibitor, Anion exchanger inhibitor |
| Lidocaine | 2-(diethylamino)-N-(2,6-dimethylphenyl)-acetamide | 234.34 | | Inhibition | Na⁺ channel blocker |
| KB-R7943 | 2-(2-(4-(4-nitrobenzyloxy)-phenyl)ethyl)isothiourea methanesulfonate | 427.50 | | Inhibition | Na⁺/Ca²⁺ exchanger inhibitor |
| SEA0400 | 2-[4-[(2,5-difluoro-phenyl)methoxy]phenoxy]-5-ethoxyaniline | 371.38 | | Inhibition | Na⁺/Ca²⁺ exchanger inhibitor |
| Benzamil | 3,5-diamino-N-[(1E)-amino(benzylamino)methyl idene]- 6-chloropyrazine-2-carboxamide | 319.75 | 10 µM | Inhibition | Na⁺/Ca²⁺ exchanger inhibitor |
| Amiloride | 3,5-diamino-6-chloro-N-(diaminomethylidene)pyraz ine-2-carbox-amide | 229.63 | 5 - 100µM | Inhibition | Na⁺/H⁺ exchanger (NHE) inhibitor |
| Nifedipine | 3,5-dimethyl 2,6-dimethyl-4-(2-nitrophenyl)-1,4-di-hydropyridine-3,5-di-carboxylate | 346.36 | | Inhibition | Ca²⁺ channel blocker |
| Angiotensin II (via AT 1 receptor) | | | 100 nM | activation | Vasoactive agent, vasoconstriction |
| Aldosterone | | | | activation | |
| Glucocorticoids | Dexamethason | 392.46 | | | |
| Catecholamines, β-adrenergic agonists α₁-adrenergic agonists | Norepinephrine, isoproterenol | | | activation | |
| cAMP and cAMP analogues | 8-Cl-cAMP; dibutyryl-cAMP | | 100 µM | activation | |
| | Rp-cAMP | | | inhibition | |
| Forskolin | [(3R,4aR,5S,6S,6aS, 10S,1 0aR,10bS)-3-ethenyl-6,10,10b-trihydroxy-3,4a,7,7,10a-pentamethyl-1-oxo-5,6,6a,8,9,10-hexahydro-2H-benzo[f]chromen-5-yl] acetate | 410.50 | | activation | |
| Cardiotonic steroids | Ouabain, rostafuroxin, bufalin, oleandrin, | | | inhibition | |
| (glycosides) | marinobufagenin, UNBS-1450 | | | | |
| Glucagon | | | | activation | |
| Insulin (via insulin receptor) | | | 200 µU/ml | inhibition | |
| | | | 400 µU/ml | | |
| Genistein | 5,7-dihydroxy-3-(4-hydroxyphenyl)chromen-4-one | 270.23 | 10 µM | inhibition | Tyrosine kinase inhibitor |
| Mitogens | Bombesin, epidermal growth factor (EGF) | | | inhibition | |
| Taurine | 2-aminoethanesulfonic acid | | 10-20 mM | inhibition | |
| Organic polycations | | | | | |
| Spermine, spermidine | N', N'-bis(3-aminopropyl)-butane-1,4-diamine, N-(3-aminopropyl)butane-1,4-diamine | | | | |
| PhTX-343 | S-N-[3-[[4-[(3-Aminopropyl)amino]butyl]-amino]propyl]-4-hydroxy-alpha-[(1-oxobutyl)amino]-benzenepropanamide tris-trifluoroacetate (Organic polycation) | | | | Derivative of the naturally occuring polyamine toxin philantotoxin |
| Agmatine | 2-(4-aminobutyl)guanidine | 130.19 | | | Polyamine in neurons |
| L-NMMA | NG-monomethyl-L-arginine | | 100 µM | inhibition | NO inhibitor |
| ODQ | 1H-[1,2,4]oxadiazolo[4,3-α]quinoxaline-1 | | 10 µM | inhibition | inhibitor of NO-sensitive guanylate cyclase |
| NOR1 | (E)-4-methyl-2-[(E)-hydoxyimino]-5-nitro-6-methoxy-3-hexenamide | | 100 µM | activation | NO donor |
| Calphostin C | 1-[3,10-dihydroxy-12-[2-(4-hydroxyphenoxy)-carbonyloxypropyl]-2,6,7,11-tetramethoxy-4,9-dioxoperylen-1-yl]propan-2-yl benzoate | 790.76 | 1 µM | inhibition | PK C inhibitor |
| Phorbol 12,13-dibutyrate | | 504.61 | 1µM | activation | PK C activator |
| cGMP and cGMP analogues | 8-bromo-cyclic GMP | | 100 µM | activation | |
| PGE₂ | (Z)-7-[(1R,2R,3R)-3-hydroxy-2-[(E,3S)-3-hydroxyoct-1-enyl]-5-oxocyclopentyl]hept-5-enoic acid | 352.47 | 100 nM | activation | |
| Indomethacin | 2-[1-(4-chlorobenzoyl)-5-methoxy-2-methylindol-3-yl]acetic acid | 357.79 | 10µM | activation | Arachidonic acid metabolism inhibitor |
| Arachidonic acid | (5Z,8Z,11Z,14Z)-icosa-5,8,11,14-tetraenoic acid | 304.47 | 30 µM | activation | |

Particularly imipramine, quinidin, cyclic adenosine monophosphate (cAMP) analogues, β-adrenergic and α₁-adrenergic agonists and the hormones angiotensin II and insulin showed strong modulating effects. It could be demonstrated that replacement of extracellular Na⁺ with N-methyl-D-glutamine (NMDG) or application of the unspecific Na⁺/Mg²⁺ exchanger inhibitors imipramine and quinidin strongly decrease SLC41A1-mediated efflux. Also, it was found that SLC41A1-mediated Mg²⁺ efflux is stimulated by db-cyclic adenosine monophosphate (db-cAMP), adrenergic receptor agonists and the hormone angiotensin II. In contrast, an inhibitory effect was observed after application of insulin.

In view of the importance of the Na⁺/Mg²⁺ exchanger activity to cell metabolism, viability and proliferation, we believe that SLC41A1 represent an ideal target for designing and /or identifying molecules that block or stimulate these processes.
Therefore, the above mentioned modulators (Table 3), especially imipramine, quinidin, DIDS, lidocaine, nifedipine, benzamil, KB-R7943, SEA0400, amiloride, agonists or antagonists of receptors for glucocorticoids, aldosterone, angiotensin II, insulin, glucagon, PGE₂ and catecholamines (β-adrenergic and/or α1-adrenergic agents), cAMP or cGMP analogues, taurine, genistein, calphostin C, phorbol 12,13-dibutyrate, L-NMMA, NOR1, ODQ, forskolin, indomethacin, arachidonic acid, spermine, spermidine, PhTX-343, agmatine, cardiotonic steroids (e.g. ouabain, rostafuroxin, bufalin, oleandrin, marinobufagenin, UNBS-1450) and of mitogens (e.g. bombesin, EGF),more preferably imipramine, quinidin, cAMP and its analogues, adrenergic agonists and molecules that mimic the effects of angiotensin II and of insulin, can be used to modulate the function, metabolism, proliferation and growth of Na⁺/Mg²⁺ exchanger expressing cells. The above named modulators are also intended for the use as therapeutic agents.

The above-mentioned antibodies, protein binding partners and the above named modulators or their analogues or agents acting as agonists or antagonists of these modulators are believed to be useful for treating patients having conditions that are characterizid by increased or decreased Mg²⁺ efflux via their Na⁺/Mg²⁺ exchanger expressing cells. Such conditions of disturbed or dysregulated Na⁺/Mg²⁺ exchanger activity have been described in various disease complexes such as cardiovascular disorders, metabolic and endocrine dysfunctions, neurologic diseases, cancer, pre-eclampsia, eclampsia, fetal growth retardation and premature labor.

Special diseases out of these disease complexes, which the present invention is focused on, are listed in Table 4. Diseases, on which the disclosure is preferably focused on, are hypertension, ischemic heart disease, myocardial infarction, cardio-vascular dysfunctions in relation to diabetes mellitus, pre-eclampsia, eclampsia, diabetes mellitus (type II), non-insulin-dependent diabetes, cystic fibrosis, morbus parkinson, stroke, prostate cancer as well as aggressive behaviour or high stress sensitivity. These diseases are marked in Table 4 (bold).

**Table 4**

| Disease complex | Disease | Role of Na⁺/Mg²⁺ exchanger | Pathogenic factors | Relevancy/strength of evidence |
|---|---|---|---|---|
| Cardiovascular | **Hypertension (high blood pressure)** | hyperactivity | Potentiation of platelet aggregation, Loss of Mg²⁺ from heart muscle cells, hepatocytes, platelets and smooth vascular muscle cells, Increased sensitivity to vasoconstrictors | ***** (5) |
| | **ischemic heart disease, myocardial infarction** | | | |
| | congestive heart failure | | | |
| | atherosclerosis | | | |
| | cardiac arrhythmias | | | |
| | sudden cardiac death | | | |
| | **Cardiovascular dysfunction in relation to diabetes mellitus** | | | |
| | **Pre-eclampsia, Eclampsia** (strongly related to pregnancy) | | | |
| Metabolism Endocrine dysfunction | **Diabetes mellitus (type I) Non-insulin-dependent diabetes (type II)** | hyperactivity | | **** (4) |
| | Hyoeraldosteronism | | | |
| | Hyperthyroidism/ hypothyroidism | | I ncrease/loss of Mg²⁺ in/from heart muscle cells | |
| | **Mukoviszidose Cystic fibrosis** | hyperactivity | | |
| Neurology/ behaviour | **Parkinson disease, Morbus Parkinson** | | | ** (2) |
| | **Aggressive behaviour, high stress sensitivity** | hyperactivity | | |
| | **stroke** | | | |
| Fetal growth retardation, premature labor | | | | ** (2) |
| cancer | **Prostate cancer** | Upregulated | | ** (2) |
| | | | | |
| | | hyperactivity | | |

In Table 4, the diseases are listed from top to bottom according to the present evidence that their pathogenesis is related to an increased or decreased Mg²⁺ efflux via Na⁺/Mg²⁺ exchanger expressing cells. The strength of evidence is indicated by stars in the last column. Cardiovascular diseases have the strongest evidence that they are related to the Na⁺/Mg²⁺ exchanger. Thus, the treatment of cardiovascular diseases is the most relevant application for the present invention, i.e. for the antibodies, the special protein binding partners as outlined above or for the specific modulators outlined above.

The nucleotide sequence of SEQ ID NO 3 (cDNA) is of special use for the gene therapy of these diseases, i.e. for the treatment of a disease from the group of cardiovascular diseases, metabolic or endocrine dysfunctions, neurological diseases, cancer, pre-eclampsia, eclampsia, fetal growth retardation and premature labor, preferably for the treatment of hypertension, ischemic heart disease, myocardial infarction, cardiac dysfunction in relation to diabetes mellitus, pre-eclampsia, eclampsia, diabetes mellitus (type I), non-insulin-dependent diabetes, mukoviszidose, cystic fibrosis (type II), parkinson disease, morbus parkinson, stroke, prostate cancer as well as aggressive behaviour or high stress sensitivity. The same applies to a fragment of SEQ ID NO 3, in which one or more nucleotides are replaced, deleted or added and which still has an identity on the nucleotide level of at least 90%.

Also disclosed herein is a model based on a non-human mammal, preferably a mouse, having an inducible conditional system leading to a functional knock out of SLC41A1, i.e. a genetically modified conditional knock out non-human mammal, which is characterized in that the SCL41A1 gene is inducible conditionally knockable (knock out), i.e. it can be knocked-out in a time (e.g., at embryonic, young or adult stage of development) or tissue-specific (particular organ or cell type) manner. In the following, the mouse is referred to because this is the preferred non-human mammal.

The mouse model was developed on the basis of the gene sequence of SLC41A1 (SEQ ID NO 3, GenBank Accession No. NM_173854).

A SLC41A1 inducible conditional KO mouse was developed by homologous recombination using 129 embryonic stem cells. This mouse line was then crossed with Cre recombinase (CR) or ligand-dependent Cre recombinase (LCR) expressing mice for the generation of tissue specific or time dependent targeted inactivation of murine *SLC41A1.*

LCR are fusion proteins between Cre and the ligand-binding domains (LBDs) of steroid receptors. The LBD has been mutated so that it does not respond to its natural ligand yet binds a synthetic ligand. As an example may serve tamoxifen-activated CreERT recombinase *(modified pacman*), a fusion of Cre with a mutated estrogen receptor (ER) LBD, that responds specifically to the synthetic drug 4-hydroxytamoxifen (*OHT*) but not to β-estradiol. In the absence of OHT, the recombinase is located in the cytoplasm. Binding of OHT to the LBD results in the translocation of the recombinase into the nucleus where it can recombine its *IoxP* substrates (Feil R., 2007). The LCR system is widely used for the generation of inducible conditional KO mice in experiments with time dependent settings but also in those with demand for tissue specificity. Mouse lines expressing CreERT2 recombinase in tissue specific manner are listed below and the newly developed conditional KO SLC41A1 mouse can be bread with one of the available CreERT2 mouse lines in dependence of specific experimental needs.

Examples of mouse lines expressing the CreERT2 recombinase in soecific tissues:
Bone (osteoblasts Col1a1-CreERT2 tg Collagen 1 α1 chain and odontoblasts)
Endothelium (Tie2-CreERT2 tg Tie2 receptor tyrosine kinase)
*Epithelium:*
Intestinal epithelium Vil-CreERT2 tg Villin el
Internal epithelial organs K18-CreERT2 tg Keratin 18
Renal epithelium KspCad-CreERT2 tg Kidney-specific cadherin Lantinga-van
Fat tissue (adipocytes) aP2-CreERT2 tg Adipocyte fatty acid binding protein
Liver (hepatocytes) SA-CreERT2 ki Serum albumin
*Nervous system:*
Astrocytes GFAP-CreERT2 tg Glial fibrillary acidic protein
GLAST-CreERT2 ki Astrocyte-specific glutamate transporter
Neural stem cells Nes-CreERT2 tg Nestin
Schwann cells and PLP-CreERT2 tg Proteolipid protein
oligodenrocytes
Schwann cells P0Cx-CreERT2 tg P0 fused to connexin 32
*Skin:*
Keratinocytes K5-CreERT2 tg Keratin 5
K14-CreERT2 tg Keratin 14
Melanocytes Tyr-CreERT2 tg Tyrosinase
Tyr-CreERT2 tg Tyrosinase
Widespread Rosa26-CreERT2 ki Rosa26.
*Muscle:*
Skeletal muscle HAS-CreERT2 tg (PAC) Skeletal muscle α-actin
Smooth muscle SM-CreERT2 ki SM22α

The transgenic conditional knock out mouse is characterized in that the genome comprises a construct, which allows conditional knock out of the SCL41A1. The term "construct" refers to a polynucleotide, which was exogenously inserted into the genom of the animal. The animal can be heterozygous or homozygous but is preferably homozygous with respect to the knock out gene (^{-/-}). The construct comprises the SCL41A1 gene of SEQ ID NO 4 (murine gene) flanked by two IoxP DNA sequence elements (IoxP sites), i.e. they are preferably situated 3' and 5' of the SCL41A1 gene.

Isolated cells from various tissues allow further investigation of the function of SCL41A1. Thus, also disclosed herein is a transgenic inducibleconditional knock out cell or cell line. Cells can be taken from the transgenic inducible conditional knock out non-human mammal or can originate from separate cell lines.

If cells from the transgenic animal are concerned, it is preferred to use primary cell cultures (primocultures) from the heart (cardiomyocytes), from the vasculature (e.g. aorta), from the liver (hepatocytes) or from the brain or CNS/microglial cells of these animals.

If cells or cell lines of other origin are concerned, it is preferred to use stable human cell lines, more preferably stable human embryonic kidney cell line (ACC-305/DSMZ) with inducible conditionally knocked out SLC41A1 gene, HEK293 (hSLC41A1^{-/-}) or stable human B-cell precursor leukemia cell line (ACC-128/DSMZ) with inducible conditionally knocked out SLC41A1 gene, NALM-6 (hSLC41A1^{-/-}). Cell lines will be generated according to the technique described by Shibahara Kei-ichi et al..
To generate inducible conditionally knocked out SLC41A1 cells or cell lines, Mammalian vectors, which comprise mutated forms of human SLC41A1 are transferred or electroporated into these primocultured cells of or into cells of the stable human cell lines, resulting in cells having in their genome mammalian vectors with a mutated human SLC41A1. The mutated forms of human SLC41A1 are as described above with respect to the mutation libraries, i.e. each of these mutated sequences can be present in the vector.

### Description of the Figures

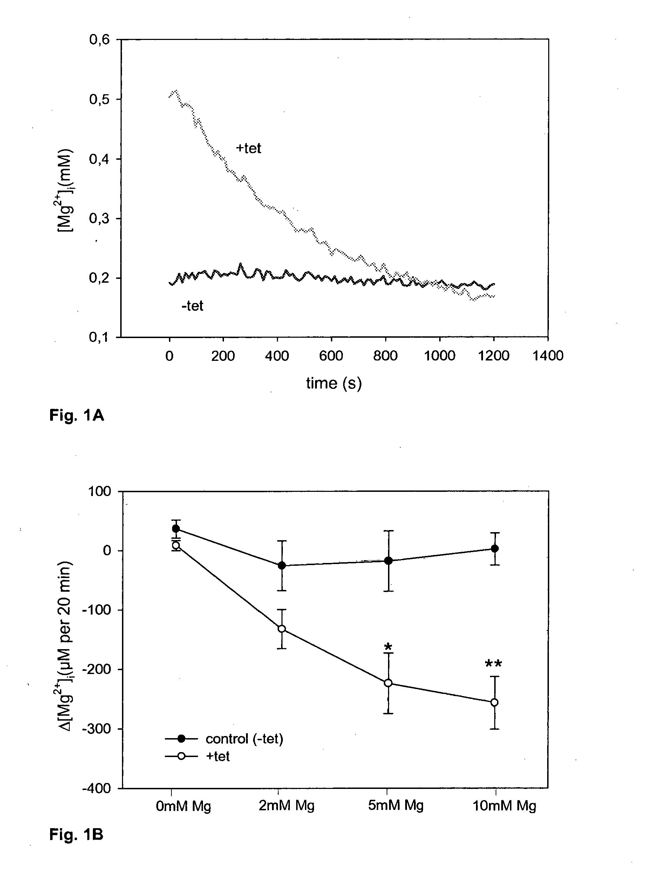

- Figure 1: shows Mg²⁺ Efflux in dependence of the Mg²⁺ concentration of the loading medium.
A: Representative original recordings of [Mg²⁺]ᵢ changes after re-suspension of -tet and +tet HEK293-(SLC41A1) cells in Mg²⁺-free, Na⁺-containing medium. Before measurements cells had been loaded with Mg²⁺ by incubating them in a high (10mM)-Mg²⁺ solution for 20 min.
B: Magnitude of mean [Mg²⁺]ᵢ changes for -tet and +tet HEK293-(SLC41A1) cells pre-loaded in solutions containing 0, 2, 5 or 10 mM Mg²⁺. N = 10 single experiments for each Mg²⁺ concentration; * P<0.01; ** P<0.001.

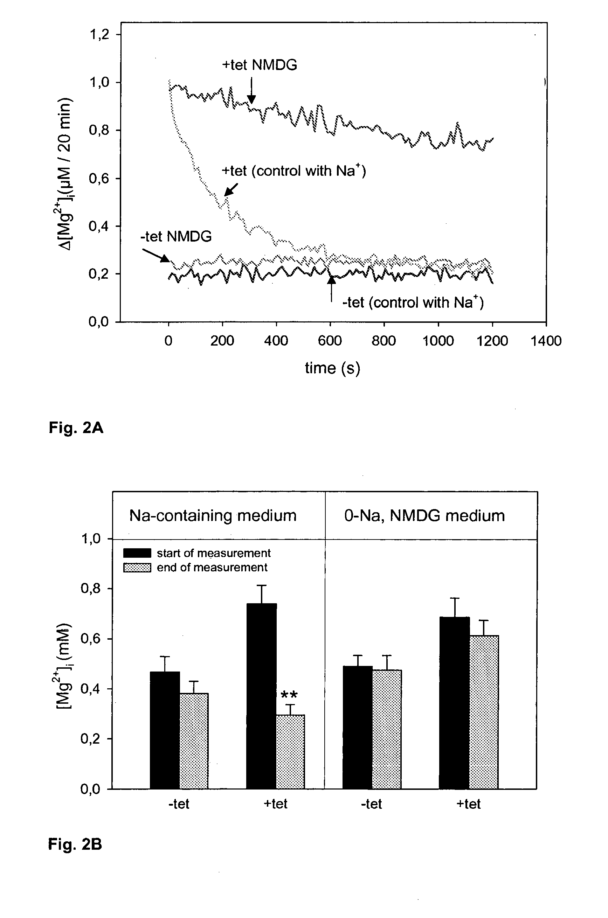

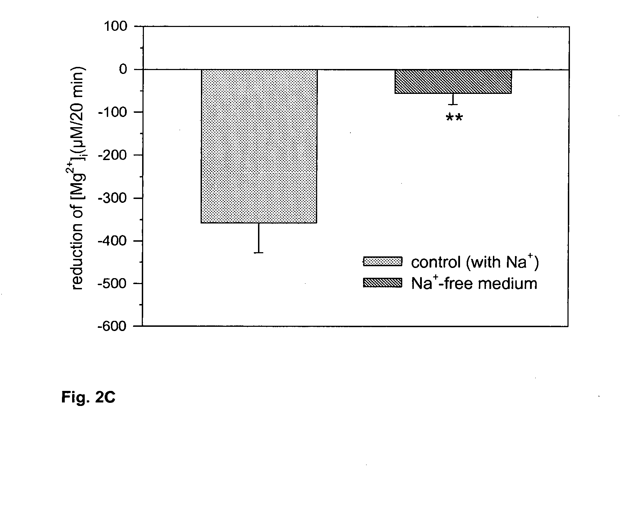

- Fig. 2: shows the effect of complete substitution of extracellular Na⁺ by NMDG on SLC41A1-dependent Mg²⁺ efflux. A: Representative original recordings of [Mg²⁺]ᵢ changes after re-suspension of -tet and +tet HEK293-(SLC41A1) cells in Mg²⁺-free, Na⁺-containing or in Mg²⁺- and Na⁺-free medium. Before measurements cells had been loaded with Mg²⁺ by incubating them in a high (10mM)-Mg²⁺ solution for 20 min. B: Effect of Na⁺substitution on [Mg²⁺]ᵢ of -tet and +tet HEK 293-(SLC41A1) cells before and after a 20 min incubation in completely Mg²⁺-free medium. N = 9 single experiments per condition; ** P<0.001. C: Effect of Na⁺ substitution on SLC41A1-dependent Mg²⁺ extrusion measured as decrease of [Mg²⁺]ᵢ. N = 9; P<0.001.Mg²⁺

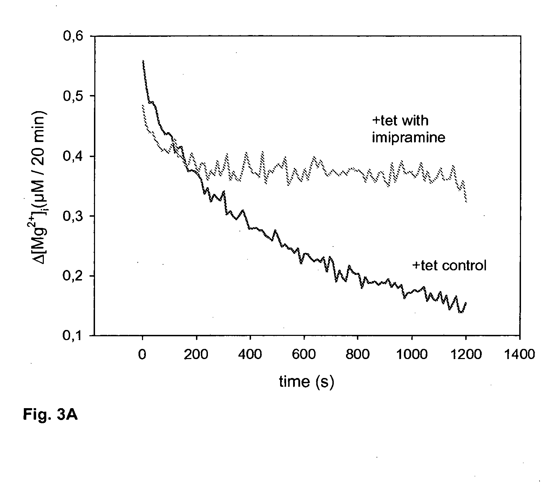

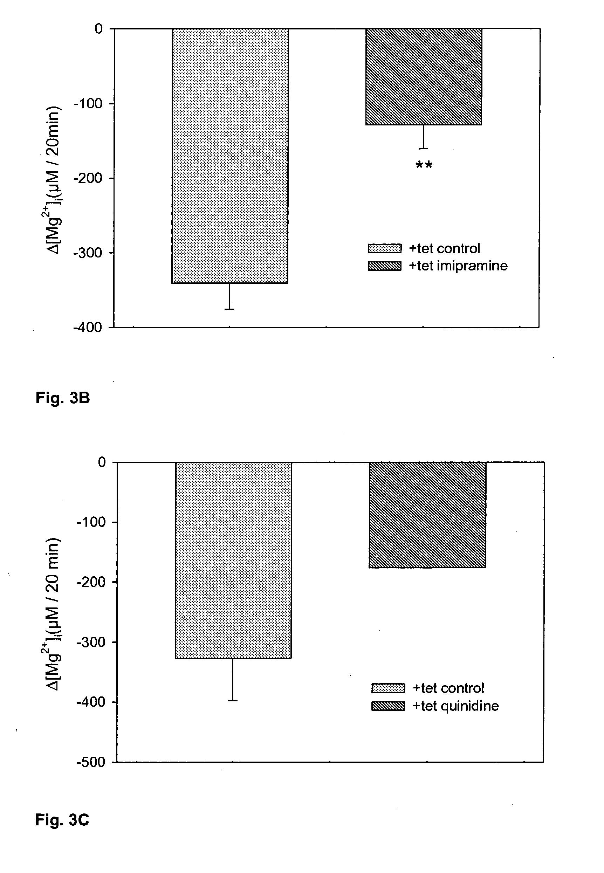

- Fig.3:: shows the effect of imipramine and of quinidin on SLC41A1-dependent Mg²⁺ efflux. A: Representative original recordings of [Mg²⁺]ᵢ changes after re-suspension of +tet HEK293-(SLC41A1) cells in Mg²⁺-free, Na⁺-containing media with or without imipramine (250 µM). Before measurements cells were loaded with Mg²⁺ by incubating them in a high (10mM)-Mg²⁺ solution for 20 min. B: Summary of the results obtained from 8 single measurements per condition. ** P<0.001. C: Summary of the effect of quinidin (100 µM) on Mg²⁺]ᵢ changes observed after re-suspension of +tet HEK293-(SLC41A1) cells in Mg²⁺-free, Na⁺-containing media. Before measurements cells were loaded with Mg²⁺ by incubating them in a high (10mM)-Mg²⁺ solution for 20 min. N = 3 single measurements per condition.

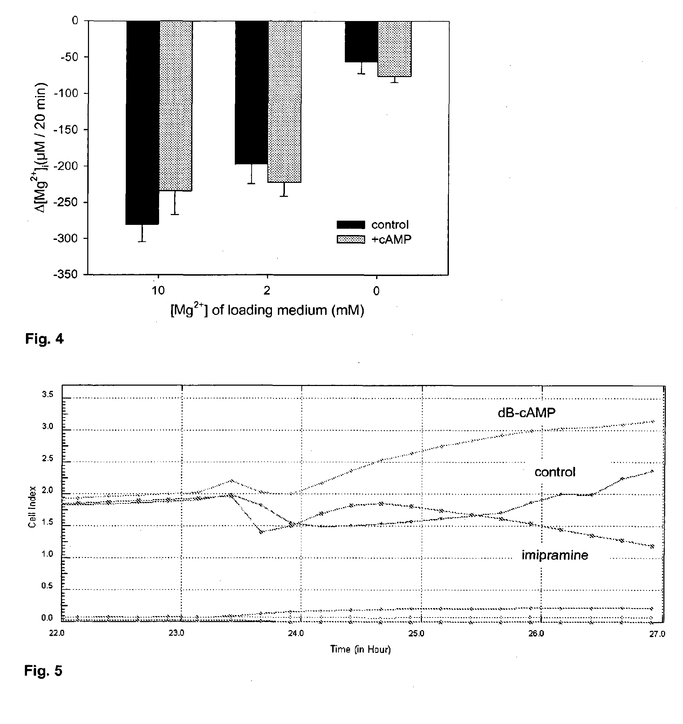

- Figure 4:: shows the effect of dB-cAMP on [Mg²⁺]ᵢ decrease in +tet HEK293-(SLC41A1) cells The [Mg²⁺]ᵢ decreased observed without and with dB-cAMP (100 µM) is shown. Bars represent means ± SE from 6 (0 mM-loading), to 10 (2 mM and 10 mM-loadingl) single measurements.
- Figure 5:: Original traces showing the effect of imipramine (250 µM) and of dB-cAMP (100 µM) on the proliferation of HEK293-(SLC41A1) cells. The respective agents are added 24 hours after seeding. Increase or decrease of the cell index reflects a higher or lower proliferation rate, respectively. Red curve: control; blue curve: imipramine; green curve = dB-cAMP.

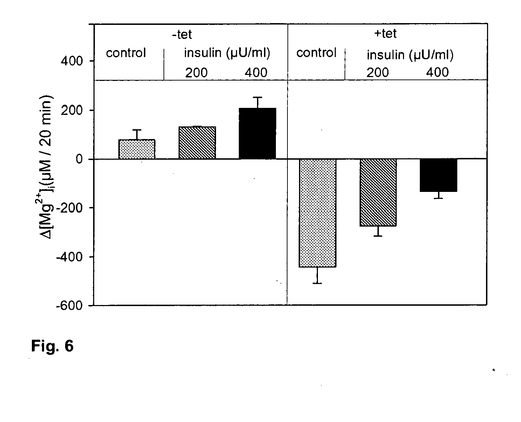

- Figure 6:: Effect of insulin on the SLC41A1-dependent Mg²⁺ efflux in -tet and +tet HEK293-(SLC41A1) cells.. [Mg²⁺]ᵢ changes after re-suspension of -tet and +tet HEK293-(SLC41A1) cells in Mg²⁺-free, Na⁺-containing media without or with insulin (200 µU/ml and 400 µU/ml) are shown. Before measurements cells were loaded with Mg²⁺ by incubating them in a high (10mM)-Mg²⁺ solution for 20 min. N = 3 single measurements per condition.

Below, the invention is outlined in more detail by means of example.

### Examples

### Material and Methods:

### HEK293-(FLAG-SLC41A1) cell line; growth media and culture conditions

Preparation (cloning protocol) and culture conditions of tetracycline-(tet)-inducible HEK293-(FLAG-SLC41A1) cell line were previously described by Kolisek et al. [2008].

### Wesfern Blot Analysis

Non-induced (-tet) and induced (+tet, 15-18 h) HEK293-(FLAG-SLC41A1) cells (107 cells ml⁻¹) were lysed for 30 min at 4 °C in Tris buffer (pH 7.5) containing 1% Triton X-100 (Bio-Rad) and protease inhibitors (Perbio-ThermoFisher). Total lysate proteins had been resolved by 10% SDS-PAGE, transferred to polyvinylidene difluoride membranes, and immunodecorated with anti-FLAG antibody coupled to HRP (Invitrogen), or with antibody to -actin (AbCam, Cambridge, UK) conjugated to GAM HRPlinked antibody (Jackson ImmunoResearch Laboratories, West Grove, PA). The same samples were immunoprecipitated by M2 anti-FLAG (Sigma) or isotype control, resolved by 10% SDS-PAGE, and transferred to polyvinylidene difluoride membranes. The membrane was immunoblotted with M2 anti-FLAG (Sigma) and GAM-_-HRP (SBA, Birmingham, AL). Membranes were developed by enhanced chemical luminescence (ECL) (Amersham Biosciences).

### Determination of free intracellular Mg²⁺ in +tet and -tet HEK293-(SLC41A1) cells by mag-fura 2 FF-Spectrofluorometry

The uninduced (-tet) and induced (+tet) HEK293-(SLC41A1) cells were rinsed twice with ice-cold, completely divalent-free Dulbecco's phosphate-buffered saline (DPBS), detached by use of Hytase (Perbio Science, Bonn, Germany), centrifuged, washed twice in completely Ca²⁺- and Mg²⁺-free Hank's balanced solution (CMF-HBS, PAN Biotech, Aidenbach, Germany) complemented with 10 mmol.l⁻¹ HEPES and 1.36 mmol.l⁻¹ L-glutamine (CMF-HBS+) and finally re-suspended in the same solution. Loading of cells with 7.5 µmol.l⁻¹ mag-fura 2 AM (Molecular Probes, Eugene, OR USA) was performed for 30 minutes at 37°C in the presence of loading facilitator Pluronic F-127 (Molecular probes). After being washed in CMF-HBS+, cells were incubated for a further 30 minuts at 37°C to allow for complete deesterification of the fluorescence probe, washed twice in CMF-HBS+ to remove extracellular mag-fura 2 and stored in CMF-HBS supplemented with 10 mmol.l⁻¹ HEPES, 5 mmol.l⁻¹ glucose and 0.4 mmol.l⁻¹ Mg²⁺ until being Mg²⁺-loaded before measurements of the Mg²⁺ efflux capacity. The latter was determined as the intracellular Mg²⁺ concentration ([Mg²⁺]ᵢ) decrease over a 20-min period. Directly before the efflux experiments, -tet and +tet HEK293-(SLC41A1) were Mg-loaded by a 20-min incubation in CMF-HBS+ containing 0, 2, 5 or 10 mmol.l⁻¹ Mg. An extracellular Mg²⁺ concentration ([Mg²⁺]ₑ) of 10 mmol.l⁻¹ was selected for all further experiments. After loading, the remaining extracellular Mg²⁺ was removed by washing the cells twice in CMF-HBS+ and, after re-suspending the cells in CMF-HBS+, measurements were started. To determine the influence of varying extracellular [Na⁺], measuring solutions were prepared by diluting the CMF-HBS+ containing 145 mM Na⁺ with appropriate volumes of a completely Na⁺- and Mg²⁺-free PBS. To investigate the effect of HCO₃⁻ on Mg²⁺ efflux, completely Ca²⁺- and Mg²⁺-free medium 199 completed with 10 mmol.l⁻¹ HEPES and 1.36 mmol.l⁻¹ L-glutamine (CMF-M199+) with Hank's (0.35 g/l NaHCO₃) or Earl's (2.2 g/l NaHCO₃) salts were used during Mg-loading and measurements. To ensure a stable pH value, the high- HCO₃⁻ media were pre-equilibrated with 95% air-5% CO₂.

Measurements were made at 37°C in 3 ml cuvettes containing 2 ml cell suspension (CMF-HBS+ or CMF-M199+ with a cytocrit of 10 %) under stirring. [Mg²⁺]ᵢ was determined by measuring the fluorescence of the probe-loaded cells in a spectrofluorometer (LS50-B, PerkinElmer Life Science, Wellesley, MA USA) by using the fast filter accessory, which allowed fluorescence to be measured at 20-ms intervals with excitation at 340 and 380 nm, and emission at 515 nm. [Mg²⁺]ᵢ values were calculated from the 340/380-nm ratio according to the formula of Grynkiewicz et al. (1981) by using the software FL WinLab version 4.0 (PerkinElmer Life Science). A dissociation constant of 1.5 mmol.l⁻¹ for the mag-fura 2/Mg²⁺ complex was used for calculations; minimum (Rₘᵢₙ) and maximum (Rₘₐₓ) ratios were determined at the end of each experiment by using digitonin. Rₘₐₓ was found by the addition of 25 mmol.l⁻¹ MgCl₂ in the absence of Ca²⁺, whereas Rₘᵢₙ was obtained by addition of 50 mmol.l⁻¹ EDTA, pH 7.2, to remove all Mg²⁺ from the solution. For data evaluation, 10-second data sets each were averaged at the beginning of the measurement and then always after 50 seconds. The final [Mg²⁺]ᵢ was determined as the mean [Mg²⁺]ᵢ of the last 10 seconds of the measurement. Thus, for the calculation of any given [Mg²⁺]ᵢ, 500 data points were used. If not otherwise stated, data are presented as means ± standard error (SE).

**Table 5: List of special media**

| **Name** | **Remarks** | **Producer** |
|---|---|---|
| -Cl⁻, -Mg²⁺, -Ca²⁺ Hank's medium | Cl⁻, Mg²⁺, Ca²⁺ free medium (Cl⁻ has been substituted with glutamate) | |
| *Completely Mg²⁺*- *and Na⁺-free PBS* | *Without Ca²⁺*/*Mg²⁺, Na⁺ substituted by N-methyl-D-glucamine (NMDG)* | *No. LZ 1825, Biochrom, Berlin, Germany* |
| Ca²⁺- *and Mg²⁺-free medium 199 Hanks* | *Without Ca²⁺*/*Mg²⁺ and phenol red* | *No. FZ 0635, Biochrom, Berlin, Germany* |
| *Ca²⁺- and Mg²⁺ -free medium 199 Earl* | *Without Ca²⁺*/*Mg²⁺ and phenol red* | *No. FZ 0615, Biochrom, Berlin, Germany* |

**Table 6: List of inhibitors**

| Name | Formula | MW (g.mol-1) | Final concentration (mol.l-1) | Producer | Inhibitor of: |
|---|---|---|---|---|---|
| Imipramine | 10,11-Dihydro-N,N-dimethyl-5H-dibenz- [b,f]azepin-5-propanamin | 280,4 | 250.10-6 | ICN Biomedica Is, Irvine, CA, USA | A, F |
| Quinidin | (2-ethenyl- 4-azabicyclo [2.2.2] oct- 5-yl)- (6-methoxyquinolin- 4-yl)-methanol | 324,4 | 100. 10-6 | | A, E |
| Verapamil | 5-[N-(3,4-dimethoxyphenylethyl)methy lamino]-2-(3,4-dimethoxyphenyl)-2-isopropylvaleronitrile hydrochloride | 491.06 | 100.10-6 | Sigma-Aldrich, St. Louis, MO USA | D |
| Niflumic acid | 2-[3-(trifluoromethyl)anilino]nicoti nic acid, 2-(α,α,α-trifluoro-m-toluidino)nicotinic acid | 282.22 | 100.10-6 | Sigma-Aldrich, St. Louis, MO USA | C |
| H₂DIDS | 4,4'-diisothiocyanatodihydrostil-bene-2,2'-disulfonic acid, disodium salt | 500.48 | 100.10-6 | Molecular Probes, Eugene, OR USA | B, C |
| NBBP | 5-nitro-2-(3-phenyl-propylamino)benzoic acid | 300.31 | 100.10-6 | Sigma-Aldrich, St. Louis, MO USA | C |

| | | | | | |
|---|---|---|---|---|---|
| A: inhibitor of Na⁺ channels and of Na⁺-dependent Mg²⁺ efflux system B: wide range chloride (anion) transporter inhibitor C: CI channel inhibitor D: L-type Ca²⁺ channels inhibitor E: K⁺ channel inhibitor F: Na⁺ channel inhibitor | | | | | |

### Results

### [Mg²⁺]ᵢ of -tet and +tet HEK293-(SLC41A1) cells.

The basal [Mg²⁺]ᵢ of Mag-Fura 2-loaded -tet and +tet (15 hrs) HEK293-(SLC41A1) cells was measured in completely Mg-free but Na-containing medium after a 20-min pre-incubation in the same solution (table 7). Compared to -tet cells (0.26 ± 0.05 mM), tetracycline-induced HEK293-(SLC41A1) cells had a 62% lower [Mg²⁺]ᵢ (0.10 ± 0.02 mM). In agreement with our former results [Kolisek et al., 2008] these data point to an increased efflux capacity of HEK293 cells over-expressing SLC41A1. To proof this, the efflux capacity of differently Mg-loaded -tet and +tet cells was determined.

### [Mg²⁺]ᵢ change in differently Mg-loaded-tet and +tet HEK293-(SLC41A1) cells.

As it is well known that the [Mg²⁺]ᵢ influences the Mg²⁺ efflux activity, the experiments were designed to support SLC41A1-related Mg²⁺ release by performing all preparation and storage procedures (with exception of probe-loading) before the actual measurements in Mg-containing solutions. In addition, directly before the efflux experiments, -tet and +tet HEK293-(SLC41A1) cells were differently Mg-loaded by a 20-min incubation in media containing 2, 5 or 10 mmol.l⁻¹ Mg. Afterwards, the Mg²⁺ efflux capacity was determined as [Mg²⁺]ᵢ decrease measured over a 20-mins period in completely Mg-free media. In table 6, [Mg²⁺]ᵢ values determined at the beginning and end of the measuring period are given for all conditions. It can be seen that -tet HEK293-(SLC41A1) cells had a stable [Mg²⁺]ᵢ of 0.25 ± 0.02 mM and no increase or decrease was observed after Mg-loading or incubation in completely Mg-free medium. In contrast, pre-incubation with increasing extracellular Mg²⁺ concentrations induced a ≈3- to 4-fold elevation of the [Mg²⁺]ᵢ in +tet HEK293-(SLC41A1) cells (table 7). When re-suspended in absolutely Mg²⁺-free media these cells show a continuous [Mg²⁺]ᵢ decrease (fig. 1A) reflecting Mg²⁺ release from the cells. Depending on loading conditions (2, 5 or 10 mM extracellular Mg) this Mg²⁺ efflux results in an 48%, 53% and 65% reduction of the endpoint [Mg²⁺]ᵢ compared to the respective starting level (Table 7). The results for -tet and +tet (15 hrs) HEK293-(SLC41A1) cells are summarized in fig. 1B. In comparison to non-induced -tet HEK293-(SLC41A1) cells, a significant Mg²⁺ extrusion occurred when +tet HEK293-(SLC41A1) cells pre-loaded with 5 or 10 mM Mg²⁺ were suspended in Mg-free medium. The [Mg²⁺]ᵢ decrease during the measuring period amounted to 133 ± 33, 224 ± 51 and 257 ± 44 µmol.l⁻¹ per 20 mins after pre-loading with 2, 5 and 10 mM of Mg, respectively (fig. 1B).

**Table 7: [Mg²⁺]ᵢ (mmol.l⁻¹) of non-induced (-tet) and induced (+tet) HEK293-(SLC41A1) cells measured after loading with various [Mg²⁺]ₑ.**

| [Mg²⁺]ₑ | -tet (control) | | +tet | |
|---|---|---|---|---|
| mM | [Mg²⁺]_{¡} at start | [Mg²⁺]ᵢ at end | [Mg²⁺]_{¡} at start | [Mg²⁺]_{¡} at end |
| 0 | 0.24 ± 0.07 | 0.28 ± 0.07 | 0.10 ± 0.02 | 0.10 ± 0.02^{A} |
| 2 | 0.23 ± 0.06 | 0.20 ± 0.06 | 0.27 ± 0.06 | 0.14 ± 0.04 |
| 5 | 0.28 ± 0.07 | 0.27 ± 0.05 | 0.43 ± 0.08** | 0.20 ± 0.04^{a} |
| 10 | 0.26 ± 0.05 | 0.27 ± 0.05 | 0.40 ± 0.04** | 0.14 ± 0.02^{b} |

| | | | | |
|---|---|---|---|---|
| [Mg²⁺]ᵢ values achieved after 20 min in loading solutions with various [Mg²⁺]ₑ of 0, 2, 5 or 10 mM ([Mg²⁺]ᵢ at start) and after 20 min in complete Mg-free media ([Mg²⁺]ᵢ at end) are given. Data are presented as means ± SE of 8 to 12 single experiments. A P < 0.05 vs. control (-tet cells); ** P < 0.001 vs. non-loaded +tet HEK293-(SLC41A1) cells ([Mg²⁺]ₑ = 0 mM); a P < 0.05 vs. control ([Mg²⁺]ᵢ of +tet cells at start), b < 0.001 vs. control ([Mg²⁺]ᵢ of +tet cells at start) | | | | |

Until now, none of the postulated Na⁺- or anion-dependent Mg²⁺ efflux systems have been identified at a molecular level. Therefore, the next step was the performance of experiments to define the transport characteristics of SLC41A1-related Mg²⁺ extrusion.

*Mg*^{*2*+} *efflux from hSLC41A1 depends on extracellular Na⁺ and is inhibited by the unspecific Na*⁺/*Mg*^{*2*+} *exchanger inhibitors imipramine and quinidine.*

First, the effect of the extracellular [Na⁺] on the Mg²⁺ extrusion was tested with Mg-loaded (10 mM) -tet and +tet HEK293-(SLC41A1) cells. The latter were suspended in absolutely Mg-free media containing 145 mM Na⁺ or in the same medium but all Na⁺ iso-osmotically replaced by the organic non-permeant cation NMDG and the [Mg²⁺]ᵢ was measured over a 20-minute period (figure 2A). In -tet control cells a steady [Mg²⁺]ᵢ of 0.45 ± 0.03 mM was determined. In contrast, the [Mg²⁺]ᵢ of +tet cells incubated in Na-containing media decreased from 0.74 ± 0.07 to 0.29 ± 0.04 mM demonstrating SLC41A1-related Mg²⁺ extrusion (fig. 2B). This Mg²⁺ efflux was clearly dependent on extracellular Na⁺ (Fig. 2). It decreased from 358 ± 69 µM/20 min in 145-Na⁺ medium to 55 ± 26 µM/20 min in 0-Na⁺NMDG medium (fig. 2C) reflecting a 82 ± 5% reduction. These results clearly point to a Na-dependent mechanism of SLC41A1-related Mg²⁺ efflux.

Therefore, we next evaluated whether application of known unspecific inhibitors of the Na⁺/Mg²⁺ exchanger resulted in changes of SLC41A1-mediated Mg²⁺ efflux. Mg²⁺-loaded (20 min, 10 mM) +tet HEK293-(SLC41A1) cells were incubated in a completely Mg²⁺-free medium with an [Na⁺]ₑ of 145 mM without or with 250 µM imipramine (Fig. 3A) or 100 µM quinidin (Fig. 3C), respectively, and the [Mg²⁺]ᵢ was measured for 20 min. To correct for molecular leakiness, control measurements were done with -tet cells. As shown in figure 3A, application of imipramine diminished the SLC41A1-related Mg²⁺ efflux. Data are summarized in figure 3B, which shows that inhibitor application reduced Mg²⁺ efflux from 341 ± 35 to 129 ± 32 µM/20min corresponding to 64 ± 9%. Figure 3C shows a summary of the inhibitory effect of 100 µM quinidin on SLC41A1-mediated Mg²⁺ efflux. It decreased from 327 ± 71 µM/20 min to 176 ± 0.5 µM/20 min corresponding to a 38 ± 7% reduction.

*hSLC41A1-mediated Mg²⁺ efflux is stimulated by dB-cAMP and angiotensin II but inhibited by insulin.*

Depending on the loading conditions and thus, on the intracellular Mg²⁺ status, application of dB-cAMP decreases (loading in 10 mM-Mg²⁺ solution) SLC41A1-related Mg²⁺ efflux by 17% or increases (loading in 2 mM- and 0 mM-Mg²⁺ loading solution) it by 13% and 36%, respectively (Figure 4). Interestingly, application of dB-cAMP to growing SLC41A1-overexpressing cells led to a stimulation of their proliferation (Figure 5). In contrast, the Na⁺/Mg²⁺ exchanger inhibitor imipramin reduced the proliferation of such cells compared to controls (Figure 5).
In addition, angiotensin II stimulated SLC41A1-related Mg²⁺ efflux by 19,5%.
As shown in figure 6, insulin reduced the SLC41A1-mediated Mg²⁺ efflux by 38% (200 µU7ml) and 68% (400 µU/ml), respectively.

*The Ca*^{*2*+} *channel inhibitor Verapamil has no effect on hSLC41A1-mediated Mg²⁺ efflux.*

In cardiac myocytes Verapamil has been show to effect Na⁺-dependent Mg²⁺ efflux by an unknown mechanism (Handy et al., 1996). Thus we tested the effect of this L-type Ca²⁺ channel inhibitor on Mg²⁺ efflux from +tet HEK293-(SLC41A1) cells. Although we used relatively high concentrations of 100 µM and 200 µM, Verapamil, respectively, we found no effect of the inhibitor on SLC41A1-mediated Mg²⁺ extrusion. The observed [Mg²⁺]ᵢ decrease amounted to 344 ± 41 µM / 20 min under control conditions and to 386 ± 45 µM / 20 min or 326 ± 58 µM / 20 min after application of 100 µM or 200 µM of verapamil.

### References

1. Feil R. (2007). Conditional somatic mutagenesis in the mouse using site-specific recombinases. In Conditional Mutagenesis: An Approach to Disease Models, eds. R. Feil and D. Metzger, Springer-Verlag, Handb Exp Pharmacol 178, 3-28.
2. Goytain A, Quamme GA. Identification and characterization of a novel mammalian Mg2+ transporter with channel-like properties. BMC Genomics 2005a; 6: 48.
3. Goytain A, Quamme, GA. Functional characterization of ACDP2 (ancient conserved domain protein), a divalent metal transporter. Physiol Genomics 2005b; 22: 382-89.
4. Goytain, A.; Quamme GA. Functional characterization of the human solute carrier, SLC41A2. Biochem Biophys Res Commun 2005c; 330: 701-5
5. Goytain A, Quamme GA. Functional characterization of human SLC41A1, a Mg2+ transporter with similarity to the procaryotic MgtE Mg2+ transporters. Physiol Genomics 2005d; 21: 337-42.
6. Goytain A, Hines, RM, El-Husseini A, Quamme GA. NIPA1 (SPG6), the basis for autosomal dominant form of hereditary spastic paraplegia, encodes a functional Mg2+ transporter. J Biol Chem 2007; 282: 8060-68.
7. GOnther T, Vormann J, Förster R (1984) Regulation of intracellular magnesium by Mg2+ efflux. Biochem Biophys Res Comm. 119: 124-131.
8. Handy RD, Gow IF, Ellis D, Flatman PW. Na-dependent regulation of intracellular free magnesium concentration in isolated rat ventricular myocytes. J Mol cell Cardiol 1996, 28: 1641-1651.
9. Kimura M (2007) Overview of magnesium nutrition. In: Nishizawa Y, Morii H, Durlach J (Eds.): New perspectives in magnesium research: nutrition and health. Springer, London, 2007, 69-93.
10. Kolisek M, Zsurka G, Samaj J, Weghuber J, Schweyen RJ, Schweigel, M. Mrs2p is an essential component of the major electrophoretic Mg2+ influx system in mitochondria. EMBO J 2003; 22: 1235-44.
11. Kolisek M, Launay P, Beck A, Sponder G, Serafini M, Brenkus M, Froschauer EM, Martens H, Fleig A, Schweigel M. (2008) SLC41A1 is a novel mammalian Mg2+ carrier. J Biol Chem; 283: 16235-47.
12. Kolisek M, Schweyen RJ, Schweigel M. (2007) Cellular Mg2+ transport and homeosasis: An overview. In: Nishizawa Y, Morii H, Durlach J (Eds.): New perspectives in magnesium research: nutrition and health. Springer, London, 2007, 21-33.
13. Kumeda Y, Inaba M. Review of magnesium and metabolic syndrome. In: Nishizawa Y, Morii H, Durlach J (Eds.): New perspectives in magnesium research: nutrition and health. Springer, London, 2007, 189-196.
14. Ödblom MP, Handy RD (1999) A novel DIDS-sensitive, anion-dependent Mg2+ efflux pathway in rat ventricular myocytes. Biochem. Biophys. Res. Comm. 264: 334-337.
15. Po SS; Wang DW, Yang IC, Johnson JP, Nie L, Bennett, PB. Modulation of HERG potassium channels by extracellular magnesium and quinidine. J Cardiovasc Pharmacol 1999, 33:181-185.
16. Quamme GA. (1989) Control of magnesium transport in the thick ascending limb. Am. J. Physiol. Renal Fluid Electrolyte Physiol. 25: F197-F210.
17. Resnick LM, Gupta RK, Laragh JH (1984) Intracellular free magnesium in erythrocytes of essential hypertension: relation to blood pressure and serum divalent cations. Proc Natl Acad Sci USA 81, 6511-6515.
18. Romanuik TL, Wang G, Holt RA; Jones SJM Marra MA, Sadar MD (2009) Identification of novel androgen-responsive genes by sequencing of LongSAGE libraries. BMC Genomics 10: 476-95.
19. Sahni J, Nelson B, Scharenberg AM. SLC41A2 encodes a plasma-membrane Mg2+ transporter. Biochem J 2007; 401: 505-13.
20. Schlingmann KP, Weber S, Peters M, Niemann-Nejsum L, Vitzhum H, Klingel K, Kratz M, Haddad E, Ristoff E, Dinour D, Syrrou M, Nielsen S, Sassen M, Waldegger S, Seyberth HW, Konrad M. Hypomagnesemia with secondary hypocalcemia is caused by mutations in TRPM6, a new member of the TRMP gene family. Nature Genetics 2002; 31: 166-70.
21. Schmitz C, Perraud AL, Johnson CO, Inabe K, Smith MK, Penner R, Kurosaki T, Fleig A, Scharenberg AM. Regulation of vertebrate cellular Mg2+-homeostasis by TRPM7. Cell 2003; 114: 191-200.
22. Schweigel M, Park H-S, Etschmann B, Martens H. Characterization of the Na+-dependent Mg2+ transport in sheep ruminal epithelial cells. Am J Physiol Gastrointest Liver Physiol 2006; 290: G56-G65.
23. Schweigel M, Park H-S, Etschmann B, Martens H. (2006) Characterization of the Na+-dependent Mg2+ transport in sheep ruminal epithelial cells. Am J Physiol Gastrointest Liver Physiol 290: G56-G65.
24. Smith, R.L., Thompson, L.J., and Maguire, M.E. (1995) J Bacteriol 177, 1233-8
25. Sponder G, Svidova S, Schweigel M, Vormann J, Kolisek M. (2010) Splice-variant 1 of the ancient domain protein 2 (ACDP2) complements the magnesium-deficient growth phenotype of Salmonella enferica sv. typhimurium strain MM281. Magnesium Research 23, 105-14.
26. Schweigel M, Vormann J, Martens H (2000) Mechanisms of Mg2+ transport in cultured rumen epithelial cells. Am J Physiol Gastrointest Liver Physiol 278: G400-G408.
27. Wabakken, T., Rian, E., Kveine, M., and Aasheim, H.C. (2003) Biochem Biophys Res Commun 306, 718-24
28. Watanabe M, Konishi M, Ohkido I, Matsufuji S. (2005) Enhanced sodium-dependent extrusion of magnesium in mutant cells established from a mouse renal tubular cell line. Am J Physiol Renal Physiol; 289, 742-48.
29. Zhou H, Clapham DE. (2009) Mammalian MagT1 and TUSC3 are required for cellular magnesium uptake and vertebrate embryonic development. Proc Natl Acad Sci U S A.: 106, 15750-5.
30. Johnsson and Varshavsky, Proc. Natl. Acad. Sci., 1994, 91 (22), 10340-10344, in U.S. patent 5,503,977 A or U.S. patent 5,585,245 A .
31. Shibahara Kei-ichi et al. in published US patent application 20100003686

## Claims

1. Mutation library for the identification or generation of SLC41A1 variants, wherein the protein SLC41A1 having the amino acid sequence of SEQ ID NO 1 is the cellular Na⁺/Mg²⁺ exchanger, comprising
i) proteins with mutations of SEQ ID NO 1, wherein one of the amino acids of SEQ ID NO 1 is substituted at positions 21 (S>N), 25 (S>Q), 73 (S>N), 76 (S>N), 80 (S>N), 89 (S>N), 97 (S>N), 158 (S>N), 309 (S>N), 394 (S>N), 408 (S>Q), 81 (T>Q), 168 (T>Q), 509 (T>Q) or 278 (Y>N) and proteins with at least one mutation of SEQ ID NO 1, wherein a plurality of these substitutions is present; wherein the mutation library consists of 15 peptides with a single aa mutation and at least one peptide wherein a plurality of these substitutions is present
or
ii) proteins with mutations of the transmembrane domains and N-, C- termini mutants of SEQ ID NO 1, wherein the amino acids 2-30, 31-60, 61-90, 2-60, 2-90 or 504-513 of SEQ ID NO 1 are deleted (6 mutated proteins)
and
proteins with mutations of SEQ ID NO 1 in membrane spanning domains, wherein the amino acids 96-205, 225-279, 286-337, 348-427 or 439-503 are deleted (5 mutated proteins)
and proteins with mutations in membrane spanning domains of SEQ ID NO 1,
wherein the amino acids 99-150, 180-241, 256-306, 317-368, 408-459 or 437-503 are deleted (6 mutated proteins); wherein the mutation library consists of 17 peptides
or
iii) proteins with leucine zipper (LZ) mutations of SEQ ID NO 1, wherein the amino acids 482-503 are deleted and wherein amino acids in LZ domain in positions 482 (L>V), 489 (L>V), 494 (L>V), 496 (L>V), 501 (L>V) or 503 (L>V) are substituted; wherein the mutation library consists of 7 peptides or
iv) proteins with external loop (EL) mutations of SEQ ID NO 1, wherein the amino acids 128-179, 247-254, 310-315, 369-405, or 468-478 are deleted; wherein the mutation library consists of 5 peptides.

## Patentansprüche

1. Mutationsbibliothek zur Identifizierung oder Erzeugung von SLC41A1 Varianten, wobei das Protein SLC41A1, das die Aminosäuresequenz mit SEQ ID NO: 1 aufweist, der zelluläre Na⁺/Mg²⁺Austauscher ist, umfassend
i) Proteine mit Mutationen der SEQ ID NO: 1, wobei eine der Aminosäuren der SEQ ID NO: 1 an den Positionen 21 (S>N), 25 (S>Q), 73 (S>N), 76 (S>N), 80 (S>N), 89 (S>N), 97 (S>N), 158 (S>N), 309 (S>N), 394 (S>N), 408 (S>Q), 81 (T>Q), 168 (T>Q), 509 (T>Q) oder 278 (Y>N) substituiert ist und Proteine mit mindestens einer Mutation der SEQ ID NO: 1, wobei eine Vielzahl dieser Substitutionen vorliegen, wobei die Mutationsbibliothek aus 15 Peptiden mit einer einzelnen As Mutation besteht und mindestens einem Peptid, bei dem eine Vielzahl dieser Mutationen vorliegt,
oder
ii) Proteine mit Mutationen der Transmembrandomänen und Mutanten der N-, C-Termini der SEQ ID NO: 1, wobei die Aminosäuren 2-30, 31-60, 61-90, 2-60, 2-90 oder 504-513 der SEQ ID NO: 1 deletiert sind (6 mutierte Proteine)
und
Proteine mit Mutationen der SEQ ID NO: 1 in membranüberspannenden Domänen, wobei die Aminosäuren 96-205, 225-279, 286-337, 348-427 oder 439-503 deletiert sind (5 mutierte Proteine)
und Proteine mit Mutationen in membranüberspannenden Domänen der SEQ ID NO: 1, wobei die Aminosäuren 99-150, 180-241, 256-306, 317-368, 408-459 oder 437-503 deletiert sind (6 mutierte Proteine), wobei die Mutationsbibliothek aus 17 Proteinen besteht,
oder
iii) Proteine mit Leucin-Zipper (LZ) Mutationen der SEQ ID NO: 1, wobei die Aminosäuren 482-503 deletiert sind und wobei Aminosäuren in der LZ Domäne an den Positionen 482 (L>V), 489 (L>V), 494 (L>V), 496 (L>V), 501 (L>V) oder 503 (L>V) substituiert sind, wobei die Mutationsbibliothek aus 7 Peptiden besteht,
oder
iv) Proteine mit Mutationen der externen Schleife (ES) der SEQ ID NO: 1, wobei die Aminosäuren 128-179, 247-254, 310-315, 369-405 oder 468-478 deletiert sind, wobei die Mutationsbibliothek aus 5 Peptiden besteht.

## Revendications

1. Banque de mutation destinée à l'identification ou à la production de variants de SLC41A1, dans laquelle la protéine SLC41A1 ayant la séquence d'acides aminés SEQ ID N°1 est l'échangeur cellulaire Na⁺/Mg²⁺, comprenant
i) des protéines présentant des mutations de la séquence SEQ ID N°1, dans lesquelles l'un des acides aminés de la SEQ ID N°1 est remplacé aux positions 21 (S>N), 25 (S>Q), 73 (S>N), 76 (S>N), 80 (S>N), 89 (S>N), 97 (S>N), 158 (S>N), 309 (S>N), 394 (S>N), 408 (S>Q), 81 (T>Q), 168 (T>Q), 509 (T>Q) ou 278 (Y>N) et des protéines présentant au moins une mutation de la SEQ ID N°1, dans lesquelles plusieurs de ces substitutions sont présentes ; dans lesquelles la banque de mutation comprend 15 peptides présentant une seule mutation aa et au moins un peptide dans lequel plusieurs de ces substitutions sont présentes,
ii) des protéines présentant des mutations au niveau des domaines transmembranaires et des mutants des extrémités terminales N et C de la SEQ ID N°1, dans lesquelles les acides aminés 2-30, 31-60, 61-90, 2-60, 2-90 ou 504-513 de la SEQ ID N°1 sont supprimés (6 protéines mutées), et
des protéines présentant des mutations de la SEQ ID N°1 au niveau des domaines transmembranaires, dans lesquelles les acides aminés 96-205, 225-279, 286-337, 348-427 ou 439-503 sont supprimés (5 protéines mutées), et
des protéines présentant des mutations de la SEQ ID N°1 au niveau des domaines transmembranaires, dans lesquelles les acides aminés 99-150, 180-241, 256-306, 317-368, 408-459 ou 437-503 sont supprimés (6 protéines mutées); dans lesquelles la banque de mutation comprend 17 peptides, ou
iii) des protéines présentant des mutations au niveau d'un domaine leucine zipper de la SEQ ID N°1, dans lesquelles les acides aminés 482-503 sont supprimés et dans lesquelles les acides aminés du domaine leucine zipper sont remplacés aux positions 482 (L>V), 489 (L>V), 494 (L>V), 496 (L>V), 501 (L>V) ou 503 (L>V); dans lesquelles la banque de mutation comprend 7 peptides,
iv) des protéines présentant des mutations au niveau de la boucle externe de la SEQ ID N°1, dans lesquelles les acides aminés 128-179, 247-254, 310-315, 369-405 ou 468-478 sont supprimés; dans lesquelles la banque de mutation comprend 5 peptides.
